# EUROPEAN PATENT APPLICATION

(11) **EP 1 693 449 A1**
(43) Date of publication of application: **23.08.2006**
(21) Application number: 04799583.2
(22) Date of filing: 10.11.2004
(51) Int. Cl.: C12N 15/09, C07K 14/47, C12N 1/15, C12N 1/19, C12N 1/21, C12N 5/00, C12Q 1/02, G01N 33/15, G01N 33/50

(54) **CELL-FREE NOTCH CLEAVAGE ANALYSIS METHOD AND DRUG SCREENING METHOD**

(30) Priority: 10.11.2003 JP 2003380562
(71) Applicant: Osaka Industrial Promotion Organization, Osaka-shi, Osaka 540-0029 (JP)
(72) Inventor: OKOCHI, Masayasu, c/o Osaka University, Suita-shi, Osaka 565-0871 (JP); TAKEDA, Masatoshi, c/o Osaka University, Suita-shi, Osaka 565-0871 (JP)
(74) Representative: Daniels, Jeffrey Nicholas
(86) International application number: PCT/JP2004/016685
(87) International publication number: WO 2005/045028

(57) **Abstract**

The present invention provides a method for analyzing cell-free Notch cleavage which can be utilized for drug screening of γ-secretase inhibitors effective for the treatment of Alzheimer's disease and the drug screening method, is the method for analyzing cell-free Notch cleavage having a cell-free cleavage reaction step of contacting a crude membrane fraction of cells in which a Notch protein mutant has been expressed with a subject substance and a detection step of detecting a fragment of an amino terminal side and a fragment of a carboxyl terminal side produced by intramembrane proteolysis (dual sequential cleavage in putative transmembrane domain) of the Notch protein mutant in the above cell free cleavage reaction step, wherein the effect of the subject substance on the intramembrane proteolysis of the Notch protein mutant is analyzed, and the Notch protein mutant has at least the putative transmembrane domain of a Notch protein, deletes a part of an amino acid sequence at the amino terminal side than at least the putative transmembrane domain and has antibody recognition sites at the amino terminal side than the putative transmembrane domain and the carboxyl terminal side, respectively.

## Description

### Technical Field

The present invention relates to analysis of γ cleavage by a cell free method and a method for drug screening, which can be utilized for drug screening of γ-secretase inhibitors and γ-secretase modifying drugs effective for treatment of Alzheimer's disease and cancer.

### Background art

The γ secretase inhibitor is extremely essential as a therapeutic drug for Alzheimer's disease. That is, an initiation of pathological process of Alzheimer's disease has been described to be the process in which amyloid β protein (Aβ) physiologically secreted out of cells is aggregated and accumulated, and it is general to think that an essence of Alzheimer's disease is the process in which this Aβ is insolubilized, accumulated and deposited.

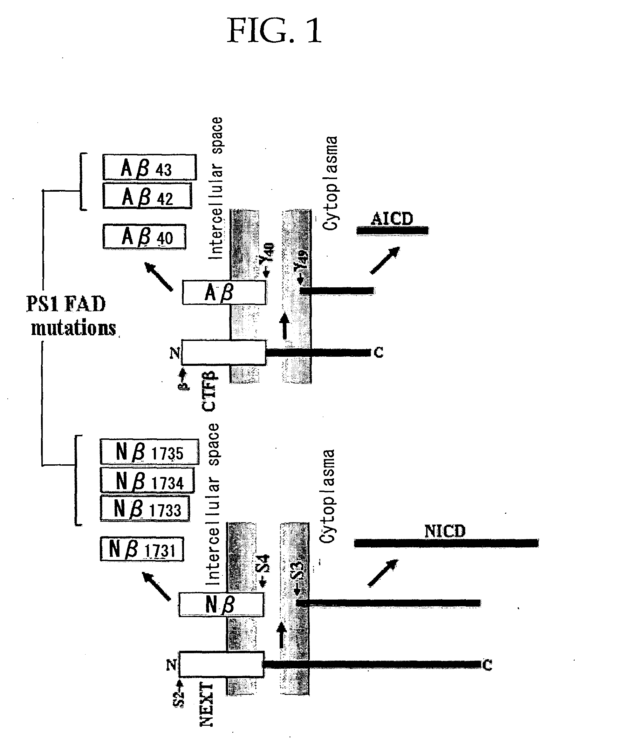

The Aβ is a peptide composed of about 40 amino acid residue generated by sequential proteolysis of β-amyloid precursor protein (βAPP) (FIG. 1). While Aβ40 of 40 amino acid residues is a major product, many subtypes such as Aβ37, Aβ38, Aβ39, Aβ40, Aβ41, Aβ42 and Aβ43 are present in small amounts due to diversity of its cleavage sites. It has been known that Aβ42 and Aβ43 in which two to three residues at the carboxyl terminus have been added are increased relative to Aβ40 by mutation of Alzheimer pathogenic presenilin, which is observed in familial Alzheimer's disease (FAD) (FIG. 1). Since these Aβ42 and Aβ43 are highly toxic and easily aggregated and accumulated, they are described to cause the familial Alzheimer's disease. Importantly, the accumulation of these Aβ42 and Aβ43 in long length is exclusively observed in pathological tissues of general sporadic Alzheimer's disease. Therefore, it is believed that medicaments which inhibit the extracellular secretion of Aβ or affect the cleavage sites of this peptide are fundamental therapeutic drugs for the Alzheimer's disease.

It has been found that the cleavage at a carboxyl terminal side which determines the toxicity of Aβ is referred to as γ cleavage and is a proteolysis by presenilin/γ secretase. In βAPP which is a transmembrane protein, shedding of an extracellular portion of the protein triggers intramembrane proteolysis dependent on presenilin/γ secretase, and in resulting fragments, one AICD (βAPP intracellular cytoplasmic domain) and the other Aβ are separated from the membrane and released in the cytoplasm and out of cells, respectively. This intramembrane proteolysis is composed of proteolysis at different two sites in the membrane ("dual-cleavage" in the membrane (γ40/γ49)).

Based on this event, the therapeutic drugs for the Alzheimer's disease have been developed by taking advantage of the inhibition of Aβ production by presenilin/γ secretase inhibitors. However, the development is deadlocked due to reports of side effects, and the drug has not come into practical use.

A system in which an Aβ amount in a supernatant of the cells in which βAPP or its mutant has been constitutively expressed is measured has been used for screening the presenilin/γ secretase inhibitors which inhibit the Aβ production. Besides, recently, a cell-free γ secretase assay system in which the production of AICD which is a carboxyl terminal fragment resulted from the proteolysis by presenilin/γ secretase is observed by culturing a membrane fraction of the cells in 37°C in which βAPP or its mutant has been constitutively expressed has been established (e.g., Non-patent Documents 1 and 2). However, when the Aβ production and the AICD production were measured in the different assay systems, it was actually impossible to measure the difference of inhibitory concentrations between the Aβ production inhibition and AICD production by the drug.

The present inventors have recently demonstrated that the same γ-secretase mechanism as in the above intramembrane proteolysis of the βAPP causes the intramembrane proteolysis of a Notch receptor protein (sometimes referred to as simply Notch or a Notch protein) (e.g., Non-patent Document 6). That is, the intramembrane proteolysis by the γ-secretase mechanism occurs in the sequential proteolysis process of Notch, Nβ (Notch-1 Aβ-like peptide) is produced as an extracellular fragment, and NICD (Notch intracellular cytoplasmic domain) which is a transcription regulatory factor including an ankyrine repeat is produced as an intracellular fragment (FIG. 1). The cleavage to produce these Nβ and NICD is composed of proteolysis at two different sites ("dual cleavage at S3 and S4 sites). Further, Nβ which is an N terminal fragment is released out of the cell by cleavage at S4. Notch is a type I transmembrane protein present on the cell surface, has an EGF repeat in the extracellular portion and has NICD (Notch intracellular cytoplasmic domain) which is the transcription regulatory factor including the ankyrine repeat in the intracellular portion.

It has been known that Notch is involved in intercellular signal transduction for cell differentiation. For example, in a developmental process of cerebral nerve system, a part of ectodermal cells differentiates into nerve precursor cells (stem cells), and further differentiates into neuron cells and glia cells. In this process, the intercellular signal transduction by Notch is important. First, Notch is expressed as a receptor on the cell which receives the Notch signal transduction. Notch transported on the cell surface is a hetero dimer by cleaving at S1 site in an extracellular region with protease such as phrine, and the dimer is kept by S-S bond on the cell surface. Subsequently, when the cells which express delta and Jagged which are Notch ligands on the cell surface and send the Notch signal are closely present, the Notch ligand and the Notch receptor interact on the cell surface to induce sequential proteolysis, and the signal transduction is initiated. That is, it is believed that Notch is cleaved at S2 site close to the cell membrane surface, further this cleavage triggers the cleavage at S3 site in the cell membrane or very close to the cell membrane in the cell, and NICD which is the fragment resulted from this event is released in the cell and directly migrates in a nucleus to control gene transcription. The above cleavage at S2 site is the extracellular cleavage by TACE (TNFα-converting enzyme), and the subsequent cleavage at S3 site is dependent on the presenilin/γ-secretase.

This way, the Notch signal transduction mechanism is extremely important for the intercellular signal transduction in the cell differentiation, and it has been reported from the recent study that Notch plays various roles such as carcinogenesis and apoptosis not only in embryo but also in adult (e.g., Non-patent Documents 3, 4 and 5). Therefore, the analysis of Notch cleavage is an important for the study on cell differentiation, carcinogenesis and apoptosis.

As described above, the present inventors have reported that Nβ which is the N terminal fragment is released out of the cell by the S4 cleavage. However, a method for analyzing intramembrane proteolysis dependent of presenilin/γ-secretase for Notch in which the NICD release and Nβ release can be analyzed simultaneously and their cleavage sites can be analyzed in detail has not been established yet.

[Non-patent Document 1] Inga Pinnix et al., THEJOURNAL OF BIOLOGICAL CHEMISTRY, Vol.276, No.1, pp.481-487, 2001

[Non-patent Document 2] CHRIS MCLENDON et al., The FASEB Journal, Vol.14, pp.2383―2386,2000

[Non-patent Document 3] Okochi, et al., "Alzheimer's Disease and Biology of Presenilin" Bunshi Seishin Igaku, Vol.1, No. 3, 2002

[Non-patent Document 4] Kageyama et al., "Control of Nerve Differentiation by Notch", Proteins, Enzymes and Nucleic Acids, Vol. 45, No. 3, 2000

[Non-patent Document 5] Brian et al., Blood Vol.96 No.5 p1906 1913 sep.12000
[Non-patent Document 6] Okochi et al., The EMBO Journal Vol.21 No.20 pp.5408-5416, 2002

### Disclosure of Invention

The present invention makes it a subject to solve various problems in the above related art and accomplish the following object. That is, it is an object of the present invention to provide a method of analyzing cell-free Notch cleavage which can be utilized for screening of γ-secretase inhibitors and γ-secretase modifying drugs effective for the treatment of Alzheimer's disease, and a method for screening the drugs.

Since presenilin/ γ-secretase is involved not only in βAPP but also in intramembrane proteolysis of 10 or more types of proteins including the Notch protein, it has been considered that the deadlock in the development of the γ-secretase inhibitors as the therapeutic drugs for Alzheimer's disease is attributed to a method of screening relied on only actions upon βAPP. For example, if the inhibitor inhibits all of these proteins, it seems that the inhibitory effect on the protein other than βAPP appears as the side effect. As suggested from that presenilin-knockout mice exhibit Notch phenotype, the action of Notch is very important for the body among these proteins. It is important to figure out the detail action of compounds upon Notch in the process of compound screening. Based on this idea, strategies to obtain data were designed.

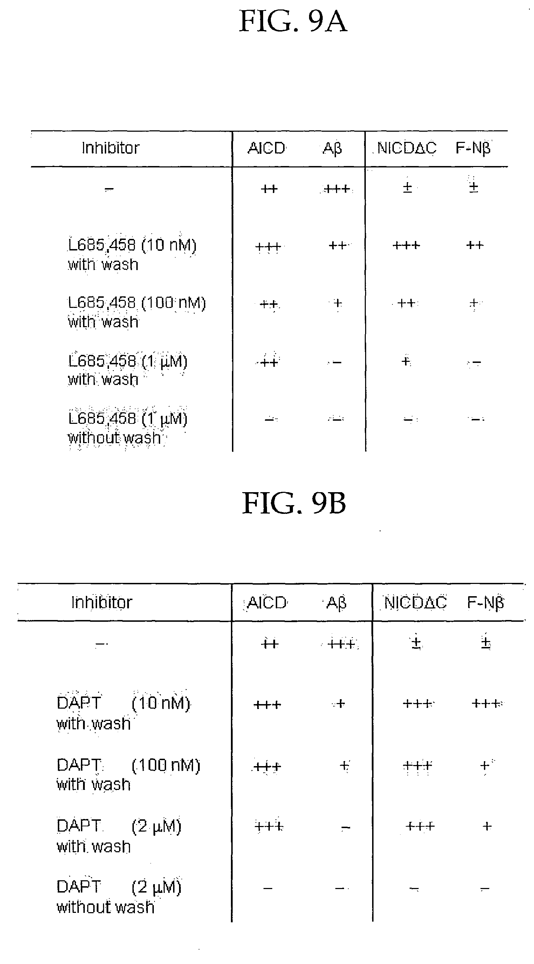

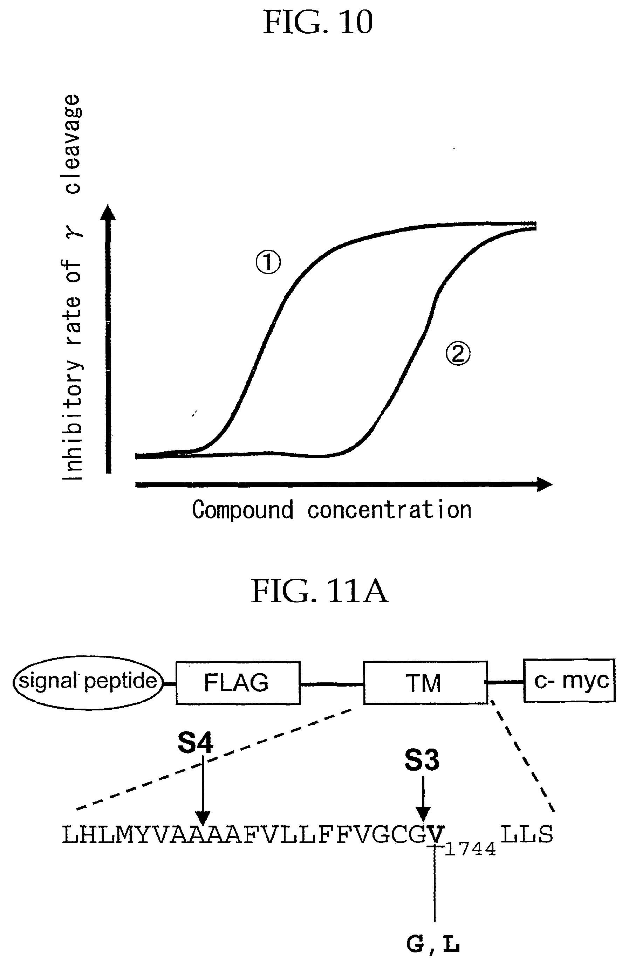

In order to solve this problem, it has been investigated that a substance which inhibits the Aβ production but does not inhibit NICD production has been examined by screening a substance which does not produce Aβ in βAPP-expressing cells and examining whether the substance inhibits the NICD production or not. However, for such two substrates, the Notch protein and Aβ, when analyzed in different assay systems, since various factors affect, it is difficult to directly compare difference of the effects of a subject substance on intramembrane proteolysis by γ-secretase, due to the difference of the substrates. The present inventors have also obtained the finding that the difference of the effect of the same drug on the intramembrane dual cleavage is larger within the substrate (e.g., between the S3 cleavage and S4 cleavage in the Notch protein) than between the substrates (e.g., between the Notch protein and βAPP) (FIGS. 9A and 9B, and FIG. 10). This fact indicates that the existing assay systems in which only one of the dual cleavage is analyzed or the dual cleavage is analyzed in the different assay systems are insufficient as the screening method for the γ-secretase inhibitors.

Another attempt has been performed where the therapeutic drugs are developed by changing precision of Aβ production and inhibiting Aβ42 production using drugs such as anti-inflammatory drugs (NSAID : non-steroidal anti-inflammatory drugs) typified by aspirin and derivatives thereof other than steroids. The present inventors have obtained the finding that there is a possibility that the γ cleavage effect on the precision of the amino terminus of NICD or AICD by γ-secretase modifying drug such as NSAID affects the signal transduction amount (FIGS. 11A to C and FIGS. 12A-1 to B). Therefore, it has been thought that it is necessary to examine the effect on ones other than the carboxyl terminus of Aβ, i.e., the amino terminus of AICD, the carboxyl terminus of Nβ and the amino terminus of NICD (mainly effects on cleavage precision) for the γ-secretase modifying drug. That is, NSAID is likely to have the side effect which the γ-secretase inhibitor does not have, and the assay system which sensitively recognizes a subtle change at cleavage site is required. In the development of the γ-secretase inhibitor and γ-secretase modifying drug which targets the Notch protein for the purpose of regulating the Notch signal, it has been also suggested that the effects on cleavage amount and cleavage precision become problems for the cleavages at S3 and S4.

Thus, the present inventor has developed F-NEXTΔC to accurately analyze the action upon Notch, and have developed a cell-free assay system which accurately reproduces the S3/S4 cleavage of Notch-1 by combining mass spectrometry and Western blotting. Furthermore, a cell-free assay system which reproduces four cleavages, γ40/γ49 cleavages of the βAPP protein and S3/S4 cleavages of Notch-1 simultaneously by further combining it with a cell-free assay of βAPP.

That is, means for solving the above subjects are as follows.
<1> A method of analyzing cell-free Notch cleavage comprising:
   a cell-free cleavage reaction step of contacting a subject substance with a crude membrane fraction of a cell in which a Notch protein mutant has been expressed; and
   a detection step of detecting a fragment of an amino terminal side and a fragment of a carboxyl terminal side resulted from intramembrane proteolysis of the Notch protein mutant in the cell-free cleavage reaction step,
   wherein an effect of the subject substance on the intramembrane proteolysis of the Notch protein mutant is analyzed, and
   the Notch protein mutant has at least a putative transmembrane domain of a Notch protein, deletes a part of an amino acid sequence in the amino terminal side than at least the transmembrane domain, and has antibody recognition sites in the amino terminal side than at least the transmembrane domain and the carboxyl terminal side, respectively.
<2> The method for analyzing the cell-free Notch cleavage according to <1>, above wherein the effect of the subject substance on an amount of the intramembrane proteolysis of the Notch protein mutant is analyzed in the detection step.
<3> The method for analyzing the cell-free Notch cleavage according to <1>, above wherein the above detection step is a step of detecting the fragment of the amino terminal side and the fragment of the carboxy terminal side using a mass spectrometry, and the effect of the subject substance on site precision of the intramembrane proteolysis of the Notch protein mutant is analyzed.
<4> The method for analyzing the cell-free Notch cleavage according to any of <1> to <3>, above wherein the crude membrane fraction is a crude membrane fraction of a cell in which the Notch protein mutant and a βAPP protein mutant have been co-expressed, the detection step further has a detection step of detecting a fragment of the amino terminal side and a fragment of the carboxyl terminal side resulted from the intramembrane proteolysis of the βAPP protein mutant in the above cell-free cleavage reaction step, wherein an effect of the subject substance on the intramembrane proteolysis of the Notch protein mutant and βAPP protein mutant is analyzed, and the βAPP protein mutant has at least a putative transmembrane domain of a βAPP protein and has antibody recognition sites at the amino terminal side than the putative transmembrane domain and the carboxyl terminal side, respectively.
<5> The method for analyzing the cell-free Notch cleavage according to any of <1> to <4>, above wherein the Notch protein mutant is either (1) or (2):
   (1) Polypeptide (FLAG-NEXT) represented by SEQ ID NO:1; 
SEQ ID NO:1;
MPRLLTPLLCLTLLPARAARGLRDYKDDDDKMVMKSEPVEPPLPS
QLHLMYVAAAAFVLLFFVGCGVLLSRKRRRQHGQLWFPEGFKVSE
ASKKKRREPLGEDSVGLKPLKNASDGALMDDNQNEWGDEDLETK
KFRFEEPVVLPDLSDQTDHRQWTQQHLDAADLRMSAMAPTPPQG
EVDADCMDVNVRGPDGFTPLMIASCSGGGLETGNSEEEEDAPAVIS
DFIYQGASLHNQTDRTGETALHLAARYSRSDRRKRLEASADANIQ
DNMGRTPLHAAVSADAQGVFQILLRNRATDLDARMHDGTTPLIL
AARLAVEGMLEDLINSHADVNAVDDLGKSALHWAAAVNNVDA
AVVLLKNGANKDIENNKEETSLFLSIRRESYETAKVLLDHFANRDIT
DHMDRLPRDIAQERMHHDIVRLLDEYNLVRSPQLHGTALGGTPTL
SPTLCSPNGYPGNLKSATQGKKARKPSTKGLACGSKEAKDLKARR
KSSQDGKGWLLDSSEQKLISEEDLEQKLISEEDLEQKLISEEDLEQKLI
SEEDLEQKLISEEDLEQKLISEEDL
   (2) A polypeptide in which tendencies of sites and amounts of the intramembrane proteolysis by γ-secretase are substantially the same as in the polypeptide (FLAG-NEXT) represented by SEQ ID NO:1.
<6> The method for analyzing the cell-free Notch cleavage according to <5>, wherein the Notch protein mutant is the above polypeptide of (2) and mass spectrometry in the Notch protein mutant is possible when the polypeptide according to (2) has been cleaved in the cell-free cleavage reaction step.
<7> The method for analyzing the cell-free Notch cleavage according to any of <5> and <6>, above wherein the Notch protein mutant is a polypeptide having a deletion of a part of an amino acid sequence at a carboxyl terminal side than the putative transmembrane domain in the polypeptide represented by SEQ ID NO:1.
<8> A method for screening drugs comprising:
   selecting an active component of the drug by using as an indicator at least any of an increase/ decrease of cleaved Notch amount and change of precision of Notch cleavage sites by a subject substance, detected by the method for analyzing the cell free-Notch cleavage according to any of <1> to <7> above.
<9> A recombinant protein comprising:
   wherein the recombinant protein is either one of a polypeptide; a polypeptide (FLAG-NEXTΔC) represented by SEQ ID NO:2, and a polypeptide composed of an amino acid sequence having deletion, substitution or addition of one or more amino acid residue in the amino acid sequence of said FLAG-NEXTΔC and where tendencies of cleavage sites and cleavage amounts by γ-secretase are substantially the same as in FLAG-NEXTΔC. 
SEQ ID NO:2;

MPRLLTPLLCLTLLPARAARGLRDYKDDDDKMVMKSEPVEPPLPS

QLHLMYVAAAAFVLLFFVGCGVLLSRKRRRQHGQLWFPEGFKVSE

AEQKLISEEDL
<10> A gene for expression comprising: wherein the gene for expression is encoding the recombinant protein according to <9> above.
<11> A recombinant vector comprising: a gene encoding the recombinant protein according to <9> above.
<12> A transformant comprising: the recombinant vector containing the gene encoding the recombinant protein according to <9> above.

According to the present invention, it is possible to provide the method for analyzing the cell-free Notch cleavage which can solve the conventional problems and can be utilized for drug screening of the γ-secretase inhibitors effective for the treatment of Alzheimer's disease, and the method for screening the drug.

### Brief Description of Drawings

FIG. 1 is a view showing sequential cleavage in a Notch protein and a βAPP protein, and illustrating that a carboxyl terminus of a polypeptide released out of a cell is changed by an Alzheimer pathogenic presenilin mutant.
FIG. 2A is a view showing that intramembrane proteolysis by presenilin/γ-secretase is given to F-NEXTΔC, i.e., F-NEXTΔC is cleaved at S4 and S3 to secret F-Nβ and NICDΔC.
FIG. 2B is a view showing how F-NEXT was modified to make F-NEXTΔC which was a Notch protein mutant. An amino acid sequence is a sequence before undergoing the cleavage of peptides released intracellularly and extracellularly.
FIG. 3A shows a procedure by homogenizing cells to prepare a crude purified membrane fraction (CMF).
FIG. 3B shows a procedure to perform a cell-free Notch cleavage assay using the crude purified membrane fraction (CMF).
FIG. 4A shows results of chase labeling experiments using a wild type PS1.
FIG. 4B shows how many molecules were released as F-Nβ in the radiolabeled molecules during 2 hours' chase after being labeled for 30 minutes.
FIG. 4C shows results of chase labeling experiments using PS1 D385N known as a dominant negative mutant of presenilin.
FIG. 4D shows results of inhibitory experiments in the case of pretreating with L685,458 for 2 hours.
FIG. 5A-1 is a spectrum in MALDI-TOF mass spectrometry of F-Nβ secreted from cells expressing F-NEXT.

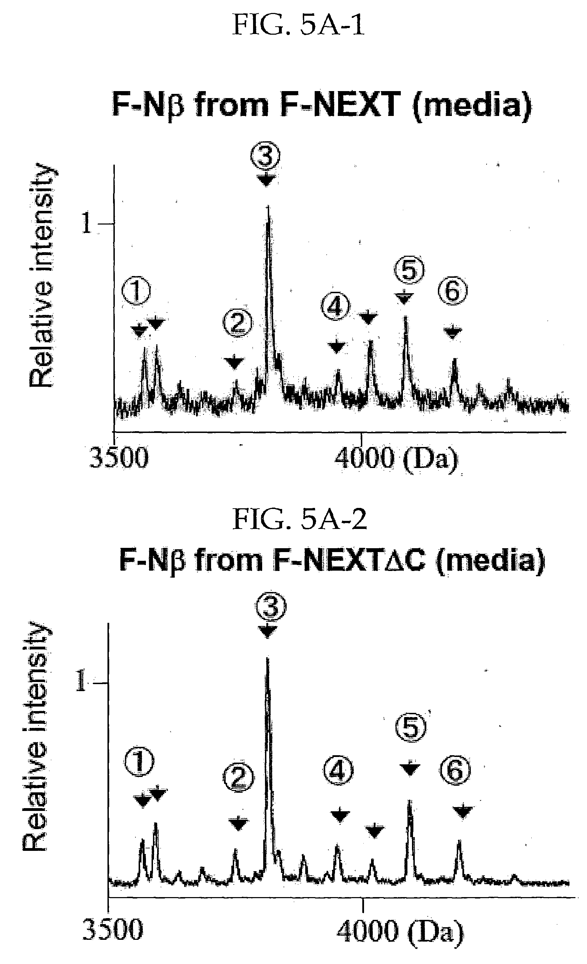

FIG. 5A-2 is a spectrum in MALDI-TOF mass spectrometry of F-Nβ secreted from cells expressing F-NEXTΔC.

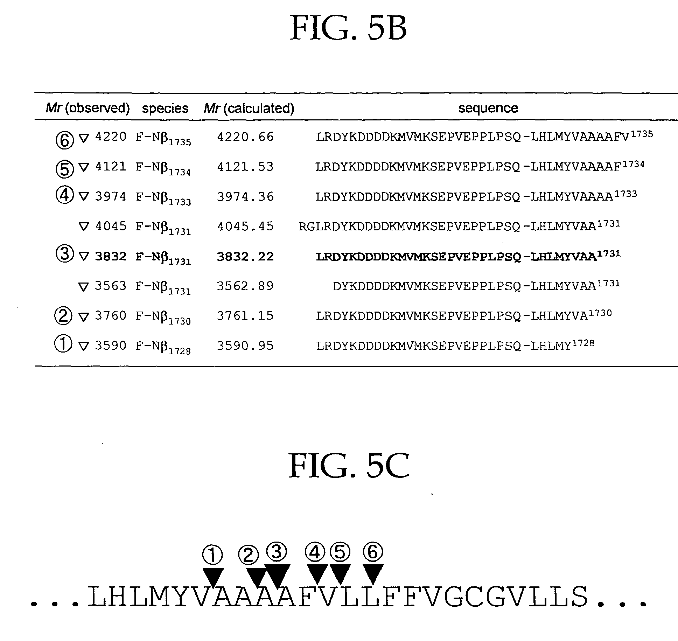

FIG. 5B lists molecular weights and peptide sequences of respective peaks.
FIG. 5C illustrates which site of cleavage releases F-Nβ in an amino acid sequence of intramembrane region of Notch-1.

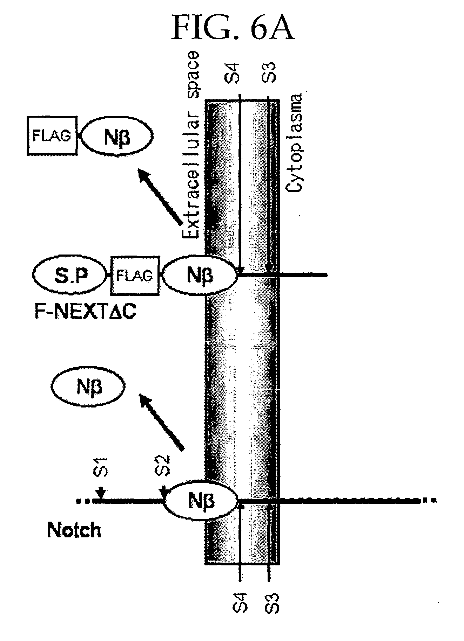

FIG. 6A is a view comparing mechanisms of sequential proteolysis in wild type Notch and F-NEXTΔC.

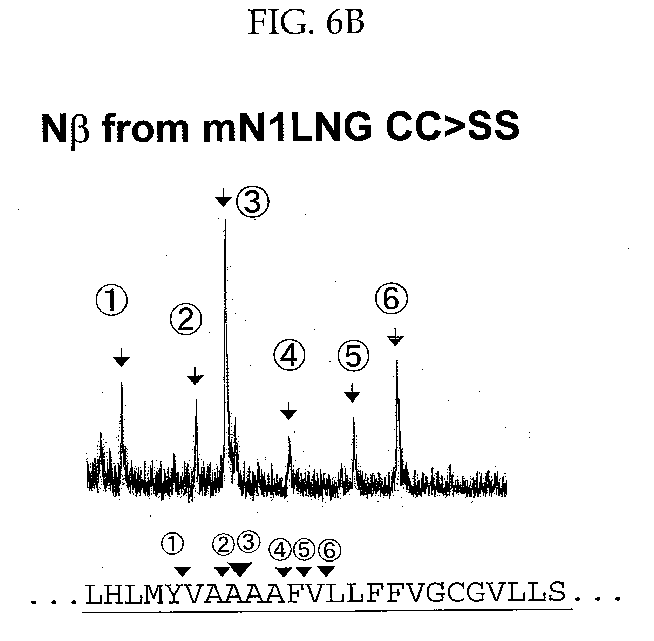

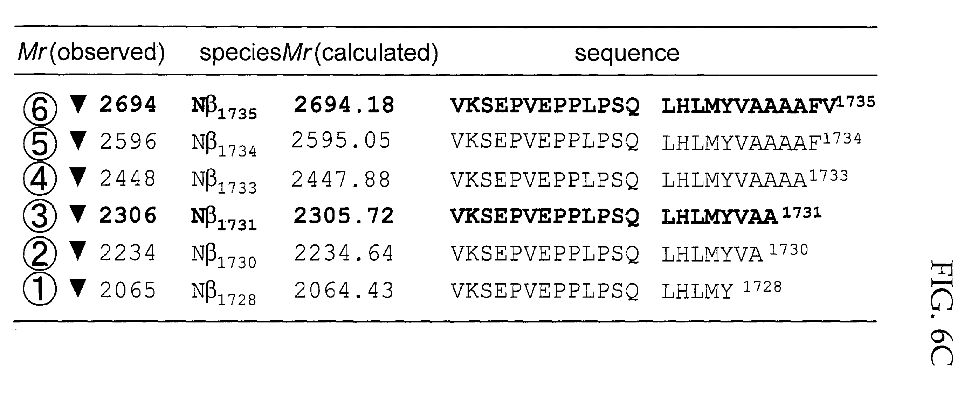

FIG. 6B shows a mass spectrum of one obtained by identifying wild type Nβ with no tag from a Notch-1 protein mutant (NILNG CC>SS) which undergoes sequential proteolysis at S1, S2 and S3 as with wild type Notch.
FIG. 6C is a view listing amino acid sequences of Nβ fragments in FIG. 6B.

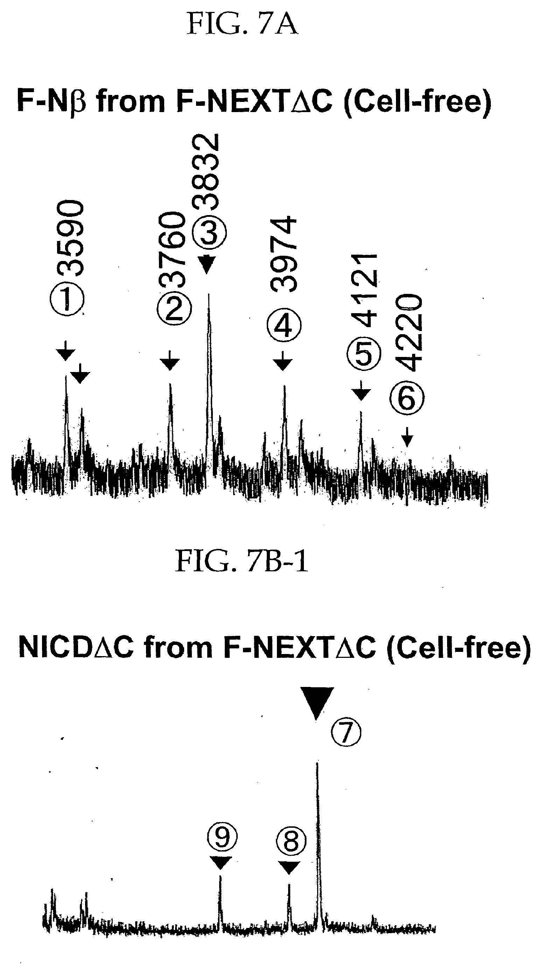

FIG. 7A shows mass spectrometric spectrum of de novo F-Nβ which is NTF produced by cell free Notch cleavage analysis.
FIG. 7B-1 shows a mass spectrometric spectrum of de novo F-NEXTΔC which is CTF resulted from cell-free Notch cleavage analysis.
FIG. 7B-2 shows amino acid sequences of peaks in FIG. 7B-1.
FIG. 7C shows S4 and S3 cleavage sites observed in FIGS 7B-1 and B-2.

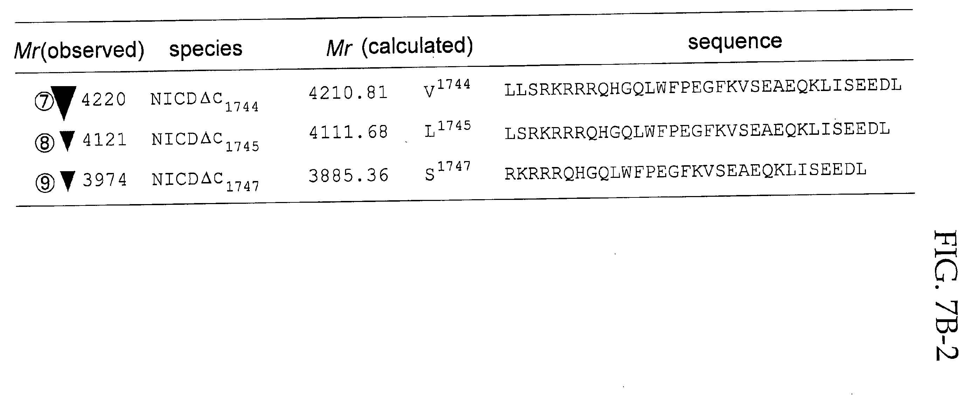

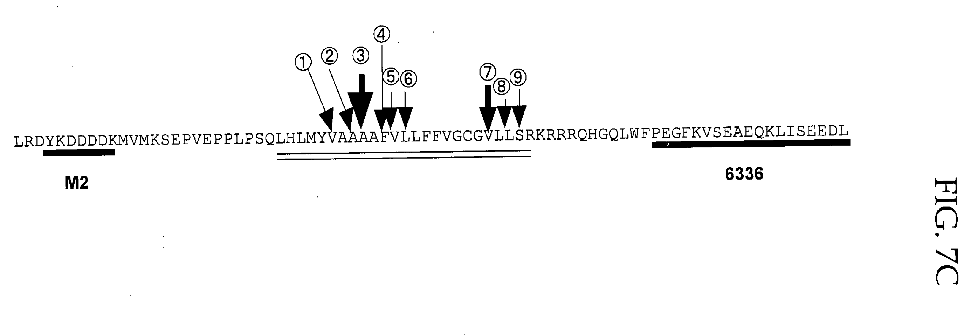

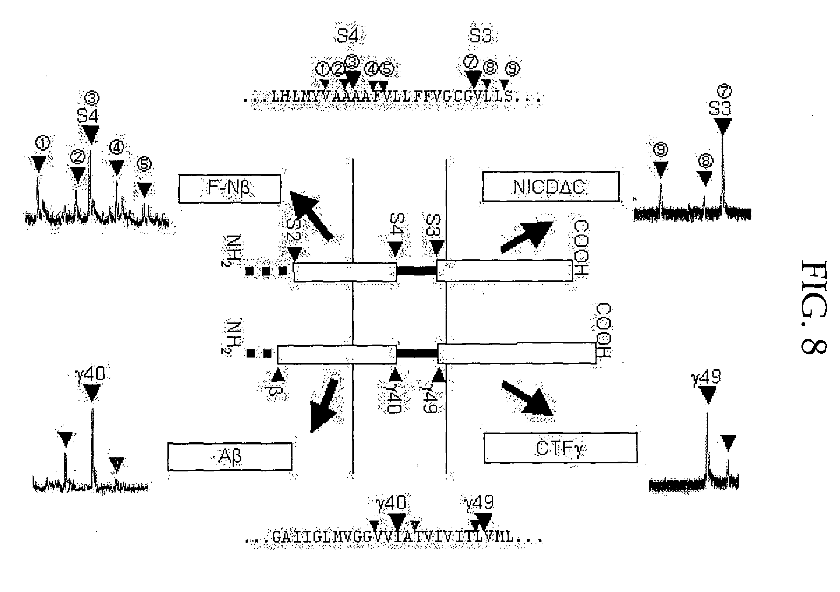

FIG. 8 shows mass spectrometric spectra of 4 fragments, Nβ, Aβ, NICDΔC and AICD observed in a multiple substrate cell-free assay system by combining βAPP and Notch-1, and cleavage sites at S4, γ40, S3 and γ49.
FIG. 9A shows results of evaluating activities of S4, γ40, S3 and γ49 cleavages on a scale of one to four when concentrations of L685,458 were changed.
FIG. 9B shows results of evaluating activities of S4, γ40, S3 and γ49 cleavages on a scale of one to four when concentrations of DAPT were changed.
FIG. 10 is a view showing inhibitory rates of γ cleavage for production of Nβ and Aβ, and AICD and NICD.
FIG. 11A is a view showing construction of F-NEXT and its mutants, F-NEXT V1744G and F-NEXT V1744L.

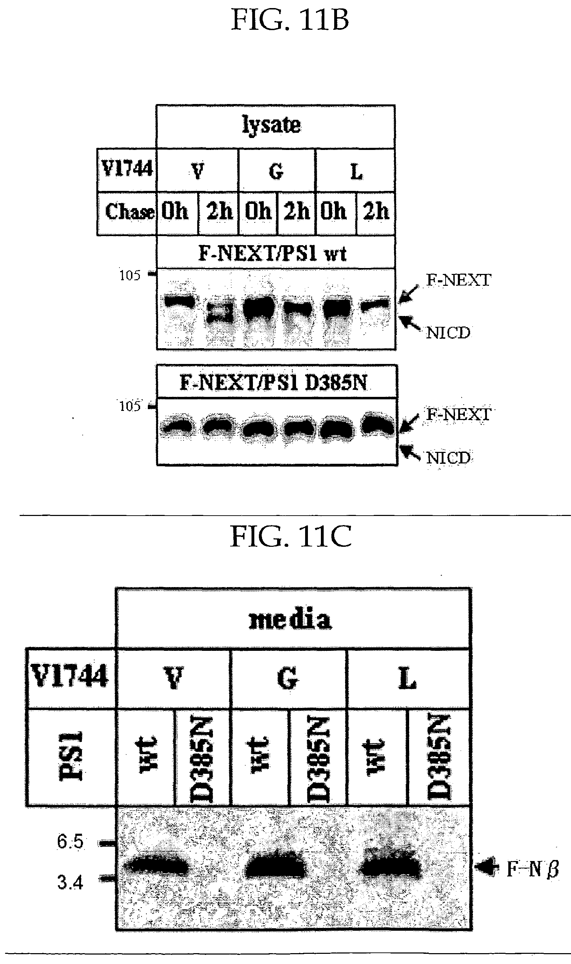

FIG. 11B is a view showing degradation of F-NEXT and its mutants, F-NEXT V1744G and F-NEXT V1744L, and production of NICD.
FIG. 11C is a view showing production of Nβ by the degradation of F-NEXT and its mutants, F-NEXT V1744G and F-NEXT V1744L.

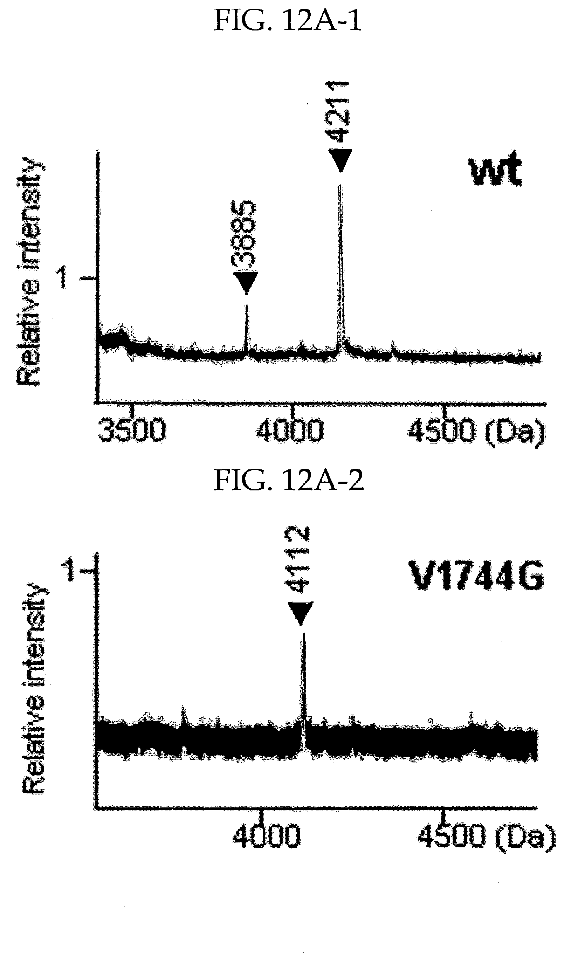

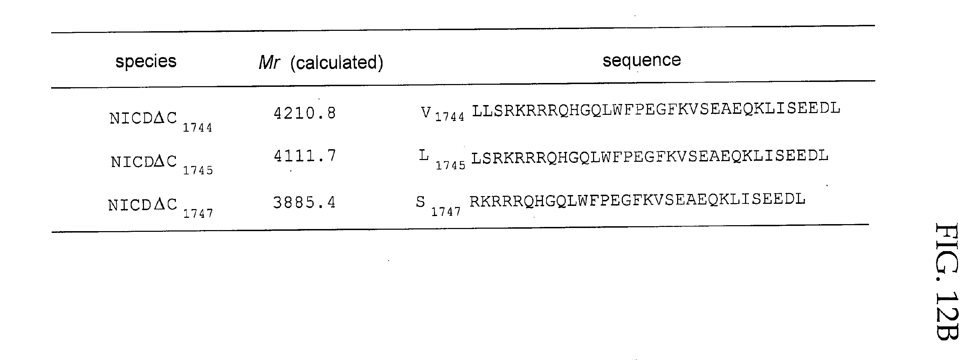

FIG. 12A-1 is a view showing results of analyzing a fragment produced by cleavage of F-NEXTΔC at S3 site by IP-MS method.
FIG. 12A-2 is a view showing results of analyzing a fragment produced by cleavage of F-NEXTΔC V1744G at S3 site by IP-MS method.
FIG. 12B is a view showing fragments produced by cleavage of F-NEXTΔC and F-NEXTΔC V1744G at S3 site.

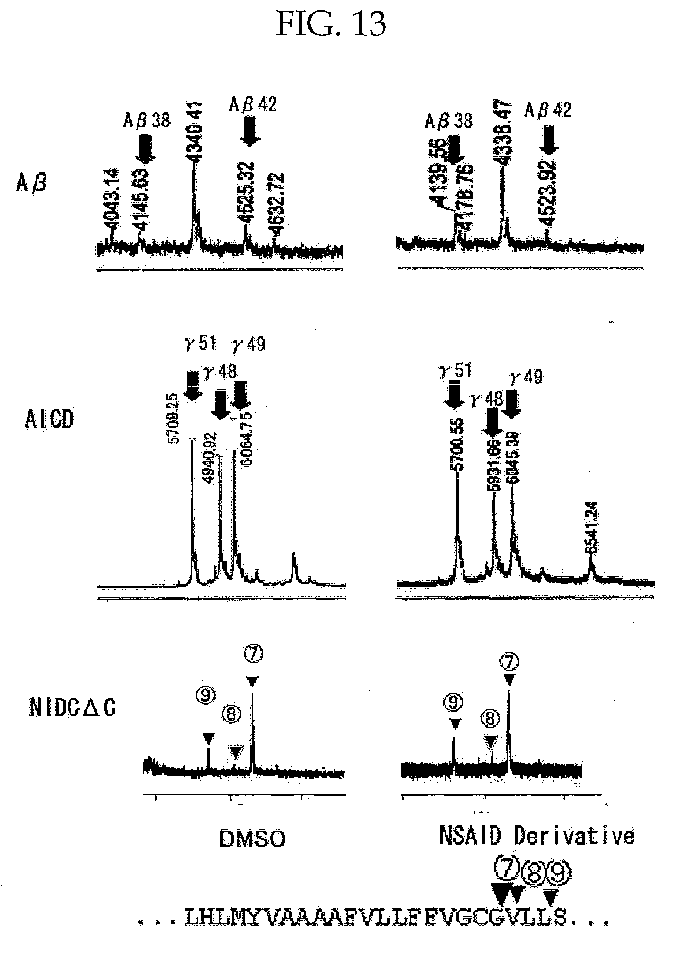

FIG. 13 is a view showing results of NSAID by cell-free Notch cleavage analysis.

### Best Modes for Carrying Out the Invention

The method for analyzing cell-free Notch cleavage of the present invention has a cell-free cleavage reaction step of contacting a subject substance with a crude membrane fraction of cells in which a Notch protein mutant has been expressed and a detection step of detecting a fragment of an amino terminal side and a fragment of a carboxyl terminal side resulted from intramembrane proteolysis of the Notch protein mutant in the above cell-free cleavage reaction step, and analyzes the effect of the subject substance on the intramembrane proteolysis of the Notch protein mutant.

The above Notch protein mutant has at least a putative transmembrane domain in the Notch protein, deletes a part of an amino acid sequence in an amino terminal side than at least the transmembrane domain, and has antibody recognition sites at the amino terminal side than the putative transmembrane domain and the carboxyl terminal side, respectively.

The Notch protein has been widely conserved over biological species, and its origin is not limited and may be human, mouse, rat, rabbit, goat, swine, cattle, Drosophila and nematode. When used for drug screening for human, it is preferable to have the amino acid sequence derived from vertebrate such as human and mouse, Notch 1 to 4 in the human and the mouse are included, and representatively, Notch-1 in the human or the mouse is included.

The putative transmembrane (TM) domain is referred to a portion presumed that the Notch protein penetrates the membrane, and is a sequence portion represented by SEQ ID NO:3 in the human Notch-1 or SEQ ID NO:4 in the mouse Notch-1. This transmembrane domain (TM) includes S3 and S4 cleavage sites, and thus the production of fragments resulted from the cleavage at these sites will be analyzed. That is, the "intramembrane proteolysis of the Notch protein mutant" is referred to two-stage cleavage at S3 and S4 in the putative transmembrane domain.
LHFMYVAAAAFVLLFFVGCGVLLS (SEQ ID NO:3)
LHLMYVAAAAFVLLFFVGCGVLLS(SEQ ID NO:4)

The above Notch protein mutant can have a tag or flag for the detection of the fragment after the cleavage as long as the tag or flag does not affect tendencies of a cleavage amount and a cleavage site. The tag and the flag is designed to be recognized by a specific antibody.

The above Notch protein mutant includes FLAG-NEXT (FIG. 2B upper column) which is a polypeptide represented by SEQ ID NO:1 and polypeptides in which the tendencies of the cleavage sites and the cleavage amount by γ-secretase are substantially the same (FIG.6B) as in a wild type Notch receptor and FLAG-NEXT. Here, NEXT (Notch extra cellular truncation) represents the polypeptide left after being cleaved at S2 site which is an extracellular cleavage site. FLAG-NEXT is the polypeptide obtained by modifying around the amino terminus of the above NEXT to add a FLAG sequence and adding a 6 time repeated c-myc sequence to the carboxyl terminus, and is designed to detect the extracellular fragment by the FLAG sequence and the intracellular fragment by the c-myc sequence. FLAG-NEXT substantially retains tendencies of the cleavage sites and the cleavage amount by γ-secretase of the wild type NEXT to which no FLAG sequence has been added (FIG. 5A-1 and FIG. 6B).

A polypeptide in which tendencies of the cleavage sites and the cleavage amount by γ-secretase are substantially the same as in the wild type Notch receptor or FLAG-NEXT is also possible to use as the Notch protein mutant of the present invention. The tendencies of the cleavage sites and the cleavage amount by γ-secretase which are substantially the same as in the wild type Notch receptor or FLAG-NEX are referred to retaining substantially the tendencies of the cleavage sites and the cleavage amount by γ-secretase (FIG. 6B) in FLAG-NEXT or NEXT to which FLAG has not been added. This is for reflecting the cleavage of the Notch protein *in vivo.* Therefore, "substantially the same" is referred to retaining the cleavage sites and cleavage amounts to an extent that the effect on Notch protein cleavage *in vivo,* required to consider the effect of the subject substance, can be estimated, and it is not necessary that the cleavage amounts and the like are completely identical. Such a polypeptide includes polypeptide having the deletion of a part of the amino acid sequence in the carboxyl terminal side than the putative transmembrane domain in the above FLAG-NEXT, and for example, includes FLAG-NEXTΔC which is the polypeptide represented by SEQ ID NO:2 (FIG. 2B lower column). FLAG-NEXTΔC is the polypeptide having the amino acid sequence obtained by deleting a majority of the NICD sequence and 5 time repeated c-myc in the 6 time repeated c-myc sequences, and hasving the tendencies of the cleavage sites and the cleavage amount by γ-secretase which are substantially the same as in FLAG-NEXT or the wild type NEXT to which no FLAG has been added. FLAG-NEXTΔC enabled the accurate analysis of the cleavage site at S3 for the first time by shortening the sequence at NICD side of FLAG-NEXT.
A base sequence of FLAG-NEXTΔC is shown in SEQ ID NO:5.

SEQ ID NO:5;
ATGCCACGGCTCCTGACGCCCTTGCTCTGCCTAACGCTGCTGCC
CGCGCTCGGCGCAAGAGGCTTGAGAGACTACAAGGACGACGA
TGACAAGATGGTGATGAAGAGTGAGCCGGTGGAGCCTCCGCTG
CCCTCGCAGCTGCACCTCATGTACGTGGCAGCGGCCGCCTTCGT
GCTCCTGTTCTTTGTGGGCTGTGGGGTGCTGCTGTCCCGCAAGC
GCCGGCGGCAGCATGGCCAGCTCTGGTTCCCTGAGGGTTTCAA
AGTGTCAGAGGCCGAGCAAAAGCTCATTTCTGAAGAGGACTTG
TAG

For the above Notch protein mutant, as long as the tendencies of the cleavage sites and the cleavage amount by γ-secretase are substantially the same as in FLAG-NEXT, in the above FLAG-NEXT and the above polypeptide obtained by shortening the FLAG-NEXT, FLAG and the antibody recognition site may be modified, and the mutation which deletes, substitutes or adds one or more amino acids residue may be included.

The cells in which the Notch protein mutant is expressed include cells transfected so that the above polypeptide is constitutively expressed. Transfection methods and host cells are not particularly limited, and the publicly known methods may be selected to use. As the host cell, for example, human embryonic kidney 293 cells (K293 cells) in which the presenilin is stably expressed can be used.

As the crude membrane fraction, it is possible to use a crude purified membrane fraction (CMF) of the cells, and the publicly known method can be optionally used for acquiring the crude purified membrane fraction. Specifically, CMF is obtained by culturing the transfected cells for a certain time period, subsequently disrupting the cells, which is then centrifuged at about 1,000 x g to yield a supernatant, and collecting a precipitated fraction by further centrifuging the supernatant at about 100,000 x g. It is preferable to add protease inhibitors which inhibit proteases other than aspartyl protease in the buffer.

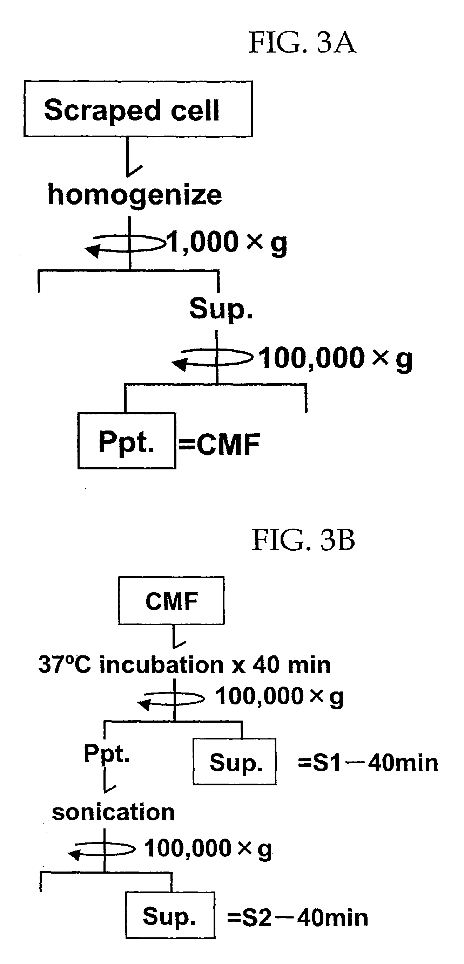

The cell-free Notch cleavage analysis is performed by thoroughly washing the above crude purified membrane fraction (CMF), suspending it in the buffer and culturing it in the presence of the subject substance at 37°C for about 40 minutes (FIG. 3B). A reaction time period can be optionally controlled. Depending on an action mechanism of a compound, the cells may be cultured in advance in the presence of the subject substance before extracting the membrane fraction. Illustrating the case of using the above FLAG-NEXTΔC as the substrate as an example, the subject substance is contacted with the crude membrane fraction acquired by the above procedure from the cells in which the FLAG-NEXTΔC has been expressed, and the reactant is centrifuged at 100,000 x g for about 15 minutes to yield a supernatant which contains an S1-40 min fraction (FIG. 3B). When the S1-40 min fraction is immunologically precipitated with a specific antibody for an intracellular portion, if the S3 cleavage has occurred, NICDΔC (i.e., corresponds to the "fragment of the carboxyl terminal side") is recognized. When a molecular weight of this NICDΔC is analyzed using an MALDI-TOF type mass spectrometry apparatus, diversity of the S3 cleavage site which is the amino terminus of NICDΔC can be analyzed. There has been no report for the diversity of this S3 sites until now, and the diversity could be demonstrated for the first time by the analysis method of the present invention.

A precipitated fraction after collecting the S1-40 min fraction is sonicated for a short time, and further centrifuged at 100,000 x g for 15 minutes to yield a supernatant, which is then collected as an S2-40 min fraction. When this S2-40 min fraction is immunologically precipitated with a specific antibody for an extracellular portion, if the S4 cleavage has occurred, Nβ (i.e., corresponds to the "fraction of the amino terminal side") is recognized. When this is analyzed by the MALDI-TOF type mass spectrometry apparatus, the diversity of the S4 cleavage site which is the carboxyl terminus of Nβ. For F-Nβ whose mass spectrometric peak is relatively small in the S2-40 min fraction, in order to examine the diversity pattern in detail, it is also possible to collect the fraction obtained by culturing the cells at 37°C overnight 12 hours.

The effect of the subject substance on the cleavage amount by γ-secretase can be detected by immunological staining, and the present invention includes any of these methods. However, the cleavage by γ-secretase is different from common proteolysis, and is accompanied by its major cleavage site and its minor cleavage site group in the vicinity which are characteristics of the intramembrane proteolysis. In order to observe the effect of the drug on the precision of this cleavage, it is necessary to use the mass spectrometry. As the mass spectrometry, publicly known methods can be used (e.g., Wang R, Sweeney D, Gandy SE, Sisodia SS. The profile of soluble amyloid beta protein in cultured cell media. Detection and quantification of amyloid beta protein and variants by immunoprecipitation-mass spectrometry. J Biol. Chem. 1996 Dec 13;271(50):31894-902). But, in order to make the accurate mass spectrometry possible, it is necessary to shorten a length of the produced fragment, and it is preferable to use the mutant such as FLAG-NEXTΔC designed so that the tendencies of the cleavage sites and the cleavage amount by γ-secretase are substantially the same as in wild type Notch or FLAG-NEXT and the length of the produced fragment is shorten to be able to perform the mass spectrometry precisely.

Advantages of using the mass spectrometry are (1) that an end of a protein fragment produced in cell-free assay can be accurately determined, (2) that simultaneously a existence rate of fragments with different end becomes quite obvious from mass spectrometric spectra, and (3) that the existence rate reflects the direct effect of the drug on "the major cleavage site and its minor cleavage site group in the vicinity which are characteristics of the intramembrane proteolysis".

The method for analyzing cell-free Notch cleavage of the present invention can be used for screening the presenilin/γ-secretase inhibitor capable of controlling Notch signaling and for various researches for Notch, and additionally can be utilized for screening of compounds in consideration of the effect on the precision in the cleavage and amount of the Notch protein with respect to the presenilin/γ-secretase inhibitor and its modifying drug effective for Alzheimer's disease.

The drug screening method of the present invention comprising: selecting an active component of the drug by using as an indicator at least any of an increase/ decrease of cleaved Notch amount and change of precision of Notch cleavage sites by a subject substance, detected by the method for analyzing cell-free Notch cleavage of the present invention. This enabled to screen the compounds in consideration of the effect on the Notch protein with respect to the presenilin/γ-secretase inhibitor effective for Alzheimer's disease.

For screening the presenilin/γ-secretase inhibitor effective for Alzheimer's disease, it is preferable to carry out the method for analyzing cell-free Notch cleavage as well as the analysis of βAPP cleavage. As the above, it is essential for the development of the γ-secretase inhibitor to accurately analyze and compare the actions of the presenilin/γ-secretase inhibitor on both protein. It is preferable to simultaneously analyze cell-free βAPP cleavage in the same system as in the method for analyzing cell-free Notch cleavage of the present invention. That is, it is preferable to be the method for analyzing cell-free Notch cleavage in which the crude membrane fraction in the above cell-free cleavage reaction step is the crude membrane fraction of cells in which the Notch protein mutant and the βAPP protein mutant have been co-expressed, the detection step further has the detection step of detecting the fragment of the amino terminal side and the fragment of the carboxyl terminal side resulted from the intramembrane proteolysis of the βAPP protein mutant in the above cell-free cleavage reaction step, and effects of the subject substance on intramembrane proteolysis of the Notch protein mutant and the βAPP protein mutant are analyzed. The βAPP protein mutant has at least the putative transmembrane domain of the βAPP protein, and has the antibody recognition sites in the amino terminal side than the putative transmembrane domain and the carboxyl terminal side, respectively. As is the case with the Notch protein mutant, for the antibody recognition site, the sequence of the protein mutant may be artificially designed to attach the tag, or the antibody specific for a native sequence may be produced.

As the method for simultaneously performed the above, it is preferable to use the membrane fraction of the cells in which the βAPP protein mutant and the Notch protein mutant have been co-expressed. As the cell which expresses βAPP, the cell transfected with Sweden type mutant βAPP (swβAPP) represented by SEQ ID NO:6 is suitably used.

SEQ ID NO:6;

































The Sweden type mutant βAPP can be obtained by introducing KM/594595/NL mutation (Sweden mutation) into a wild type gene cloned from human cDNA library. The Sweden mutation can be introduced by a publicly known 2 step PCR method. The same procedure as in the case of the Notch protein can be used for acquisition of the membrane fraction and the detection of the fragment cleaved by γ cleavage. In βAPP, the sequence in the carboxyl terminal side than the putative transmembrane domain is originally short, and the cleavage site can be detected by mass spectrometry even when the protein is not shortly modified.
SEQ ID NO:7
GAIIGLMVGGVVIATVIVITLVML

In the analysis method of the present invention, the accurate analysis is possible by using the cell-free system because the degradation of protein fragments produced by the intramembrane proteolysis does not occur. It is advantageous in that the screening is widely possible even when the leading componud is toxic. Such a toxicity can be eliminated by subsequent optimization and the such a compound becomes a useful drug in some cases.

The recombinant protein of the present invention is a polypeptide (FLAG-NEXTΔC) represented by SEQ ID NO:2; or a polypeptide composed of an amino acid sequence having deletion, substitution or addition of one or more amino acid residue in the amino acid sequence of said FLAG-NEXTΔC and where tendencies of cleavage sites and cleavage amounts by γ-secretase are substantially the same as in FLAG-NEXTΔC. As aforementioned, these proteins are preferably used in the method for analyzing cell-free Notch cleavage of the present invention.

The gene for expression of the present invention is characterized by encoding the above recombinant protein, and is introduced into a recombinant vector to introduce into a host cell in order to produce the membrane fraction used for the method for analyzing cell-free Notch cleavage of the present invention.

The recombinant vector of the present invention is not particularly limited as long as it contains the gene encoding the recombinant protein of the present invention.

For a transformant of the present invention, an introduction method and the host cell are not particularly limited as long as it contains the recombinant vector containing a gene encoding the recombinant protein of the present invention and forcibly expresses the recombinant protein of the present invention. As the host cell, the cell which stably expresses the presenilin is preferable, and for example, human embryonic kidney 293 cell (K293 cell) and the like are included. Such a transformant can be used for the method for analyzing cell-free Notch cleavage of the present invention.

### Example

Examples of the present invention will be described below, but the present invention is not limited to these Examples. Reagents, materials and experimental procedures in Examples are as follows.

### (Reagents)

L685,458 and DAPT which are γ-secretase inhibitors, and PMA which is phorbol-12-myristate-13-acetate were purchased from Calbiochem.

### (Cultured cells and cell lines)

Human embryonic kidney 293 cells (K293 cells) were cultured in DMEM medium supplemented with 10% fetal calf serum, 1% penicillin/streptomycin, 500 µg/ml of neomycin (for selecting βAPP expression), 200 µg/ml of zeocin (for selecting PS1 expression) and 100 µg/ml of hygromycin (for selecting F-NEXTΔC and F-NEXT expression).

The cells were transfected with βAPP, F-NEXTΔC, mNILNG, CC>SS or F-NEXT using a brand name Lipofectamine 2000 (Invitrogen). After the selection with antibiotics for about 20 days, a cell line in which the transfected protein had been stably expressed was isolated and cultured to use as a single cell clone, or a pooled cell clone obtained by mixing several ten single cell clones were used for the experiments. K293 cells in which PS1, wt or PS1 D385N had been stably expressed were made by Okochi's and Steiner's methods (Okochi et al, 2000, Steiner et al,1999).

### (Pulse-chase experiment)

In order to determine whether an N terminal fragment (NTF: F-Nβ) was released from the cells which had expressed F-NEXTΔC or F-NEXT as a result of proteolysis by presenilin/γ-secretase, the cells (K293 cells) which had stably expressed F-NEXTΔC or F-NEXT were cultured in a 10 cm dish up to a confluent phase. The cells were cultured in DMEM solution deprived of methionine (obtained by modifying a commercially available one from Gibco) for 45 minutes to be starved with methionine. Subsequently, the medium was replaced with the same DMEM solution deprived of methionine, and simultaneously the cells were metabolically pulse-labeled with 300 µCi of [³⁵S]-methionine/cysteine (Pro-mix, Amersham) for 30 minutes. Then the cells were chased in 10% FCS/DMEM for 2 hours.

### (Immunoprecipitation/SDS-PAGE in pulse chase experiment)

After termination of a chase period, the medium was collected and immediately placed on ice. Then cell debris was eliminated by centrifuging at 3,000 x g. Subsequently, a protease inhibitor cocktail (1:1000, Sigma) and 0.025% sodium azide were added. The sample was immunoprecipitated with 4G8 antibody or anti-FLAG-M2-agarose (Sigma) overnight, and a precipitate was washed three times with RIPA buffer containing 0.1% SDS, 0.5% deoxycholic acid and 1% Triton X-100. Subsequently, SDS-PAGE was performed using 10 to 20% gradient gel of Tris-tricine (Invitrogen). The cells were collected in ice-cooled PBS, separated from the medium by centrifugation at 1,500 x g, and lysed in 100 µl of the RIPA at 10 time concentration. And, 900 µl of PBS containing a protease inhibitor mixture (1:500, Sigma) was added to the lysed cells.

An insoluble fraction was centrifuged at 15,000 x g, and its supernatant (RIPA soluble fraction) was used for an immunoprecipitation reaction. The sample for the immunoprecipitation was pretreated with protein A Sepharose (Sigma), and immunoprecipitated with 6336, 9E10, 6613 or M2 agarose.

Subsequently, washed protein samples were separated by 8% or Tris-tricine SDS-PAGE. After fixing the gel, the gel was shaken in an amplify fluorographic reagent (Amersham), then dried, and finally autoradiography was performed.

### (Immunoprecipitation/MALDI-TOF MS analysis)

In the case of the culture supernatant, the cells which had stably expressed F-NEXT or its derivative were cultured in a 20 cm dish up to the confluent phase, and then the culture medium was replaced with new 10% FCS DMEM. The cells were cultured in a CO₂ incubator for 3 hours, then the culture supernatant was collected and immediately placed on ice, and subsequently the cell debris was eliminated by centrifugation. After adding 50 mM Tris buffer (pH 7.6), 5 mM EDTA, the protease inhibitor mixture (1:1000, Sigma) and 0.025% sodium azide, the medium was immunoprecipitated at 4°C for 4 hours using 4G8 or M2 agarose.

In the case of cell-free γ-secretase assay, MS washing buffer composed of 4 time amount or more of 0.1% n-octylglucoside, 140 mM NaCl, 10 mM Tris (pH 8.0) and 0.025% sodium azide was added to an S1 or S2 fraction which was the supernatant of ultracentrifugation after the assay. The mixture was immunoprecipitated at 4°C for 4 hours using 6636, 6618, 4G8 or M2 agarose. After the immunoprecipitation, the precipitate was washed three times at 4°C for 10 minutes using the MS washing buffer. The precipitate was washed once more with 10 mM Tris (pH 8.0) containing 0.025% sodium azide, and then simply washed once more with water. A peptide bound to the specific antibody in the precipitate consequently obtained was eluted with TFA/acetonitrile/water (TFA : acetonitrile : water = 1:20:20) saturated with α-cyano-4-hydroxy cinnamic acid. The solubilized sample was dried on a stainless plate, and analyzed by MALDI-TOF MS. MS peaks were calibrated by angiotensin (Sigma) and insulin β chain (Sigma).

### (Example 1)

The S1, S2, S3 and S4 sites have been identified as sequential proteolysis sites of the Notch-1 protein. The proteolysis at S1 site and the subsequent formation of a heterodimer of the Notch protein on the membrane surface appear to be essential modifications for Notch to function as the receptor. When this Notch receptor is bound to a Notch ligand expressed on an adjacent cell, the Notch protein is further cleaved at S2 site by taking this opportunity (extracellular shedding). A carboxyl terminal fragment cleaved at S2 site is referred to as NEXT (Notch extra cellular truncation). NEXT becomes a substrate of the presenilin/γ-secretase, and undergoes the intramembrane proteolysis to release Notch-β and NICD extracellularly and intracellularly, respectively. Functional analysis of NICD has advanced, and it has been demonstrated that NICD directly migrates in the nucleus and functions as a signal transduction molecule.

The present inventors have made a mutant analogue of NEXT in order to directly, sensitively and accurately observe the proteolysis of the Notch protein by the presenilin/γ-secretase.

### -Development of F-NEXTΔC-

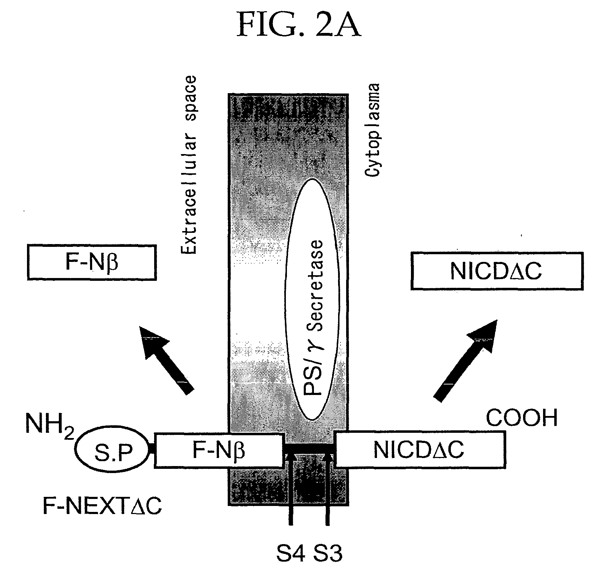

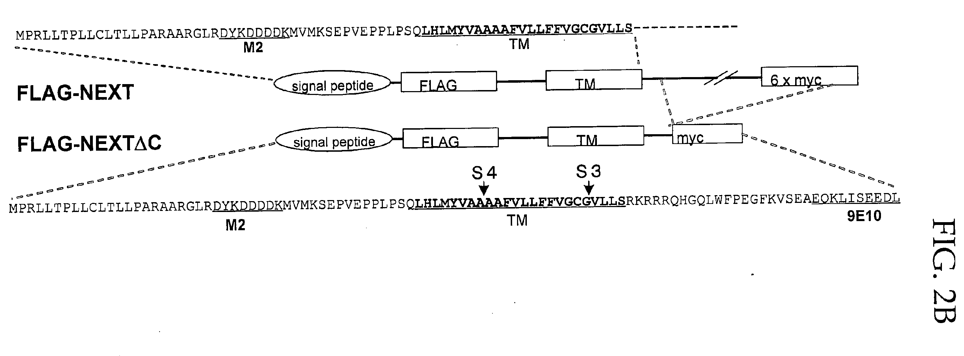

F-NEXTΔC suitable for investigating details of the intramembrane proteolysis of the Notch protein was made (FIG. 2A). Configurations of FLAG-NEXT (F-NEXT) and FLAG-NEXTΔC (F-NEXTΔC) are shown in FIG. 2B.

### (Plasmid)

A plasmid for expression in animal cells incorporating a cDNA encoding NEXT (6 time repeated c-myc sequence had been added to its C terminus) in which FLAG sequence had been added to its N terminus, i.e., FLAG-NEXT (F-NEXT) into pcDNA3(Hygro) was prepared by Okochi et al's method (Okochi, M., Steiner, H., Fukumori, A., Tanii, H., Tomita, T., Tanaka, T., Iwatsubo, T., Kudo, T., Takeda, M., Haass, C. Presenilins mediate a dual intramembranous gamma-secretase cleavage of Notch-1. EMBO J (2002) 21, 5408-5416.).

An expression plasmid for F-NEXTΔC obtained by drastically removing the amino acid sequence in the intracellular portion from F-NEXT was produced using a site-directed mutagenesis kit (ExSite PCR-Based Site-Directed Mutagenesis Kit, Stratagene) and using F-NEXT as a template. At that time, the following two primers 1 and 2 (SEQ ID NOS:8 and 9) were prepared.

Primer-1:

5'-p-gag-caa-aag-ctc-att-tct-gaa-gag-gac-ttg-tag-tcc-tgc-agc-ccg-ggg-

gat-cca-c-3 (55 mers) (SEQ ID NO:8)

Primer-2:
5'-p-ggc-ctc-tga-cac-ttt-gaa-acc-ctc-agg-gaa-cca-gag-ctg-gcc-3' (42 mers)
(SEQ ID NO:9)

It was confirmed by sequencing the nucleotide sequence of this mutant that the mutagenesis had succeeded.

Compared with F-NEXT, in F-NEXTΔC the NICD portion which is released in the cell and migrates in the nucleus after the intramembrane proteolysis is drastically eliminated. Furthermore, the artificial 6 time repeated c-myc tag added to the carboxyl terminus in F-NEXT was also reduced to one c-myc tag. This construct is designed so that F-Nβ extracellularly secreted by the presenilin/γ-secretase is recognized by anti-FLAG tag antibody (M2 agarose) and NICDΔC intracellularly released is recognized by 6336 antiserum designed to specifically identify it. The carboxyl terminal fragment is designed to shorten from NICD to NICDΔC for (1) applying NICDΔC to the immunoprecipitation and the mass spectrometry system to accurately identify the cleavage site by the presenilin/γ-secretase which directly produces NICD, and also in consideration of (2) preventing NICD from migrating into the nucleus to exert the function.

### (Antibody)

Since the intracellular portion of NICDΔC expressed in the cells was difficult to be recognized by the anti-c-myc antibody, rabbit antiserum for efficiently recognizing and identifying this was made. The polyclonal antibody (6336) is the antibody against the following synthetic polypeptide (SEQ ID NO:10). First, the polypeptide which became an antigen was prepared. A KLH protein was chemically crosslinked to a Cys residue at the amino terminus of the polypeptide to make the antigen.

Besides, antiserum which recognized specifically mouse Nβ was made. The polyclonal antibody (6521) was obtained by chemically crosslinking the KLH protein to the Cys residue at the carboxyl terminus of the following polypeptide (SEQ ID NO:11) to use as the antigen.

Anti-c-myc monoclonal antibody (9E10) was used to recognize the 6 time repeated c-myc sequence, and M2 agarose obtained by covalently binding anti-FLAG monoclonal antibody to agarose was used for recognition of the FLAG sequence. 4G8 was purchased from Senetec.

SEQ ID NO:10:

NH2-CEGFKVSEAEQKLISEEDL-COOH

SEQ ID NO:11:

NH2-VKSEPVEPPLPSC-COOH

### -Preparation of membrane fraction-

It was investigated in detail whether the intramembrane proteolysis by the presenilin/γ-secretase which occurred in F-NEXTΔC was identical to that which occurred in F-NEXT.

First, in Examples 2 and 3, natures and site difference in the cleavage of the carboxyl terminus in the amino terminal fragment (NTF) extracellularly secreted were investigated.

### (Example 2)

It was demonstrated that a type and an amount of F-Nβ secreted from the cells which had expressed F-NEXTΔC were not different from those secreted from the cells which had expressed F-NEXT (*in vivo* experiment).

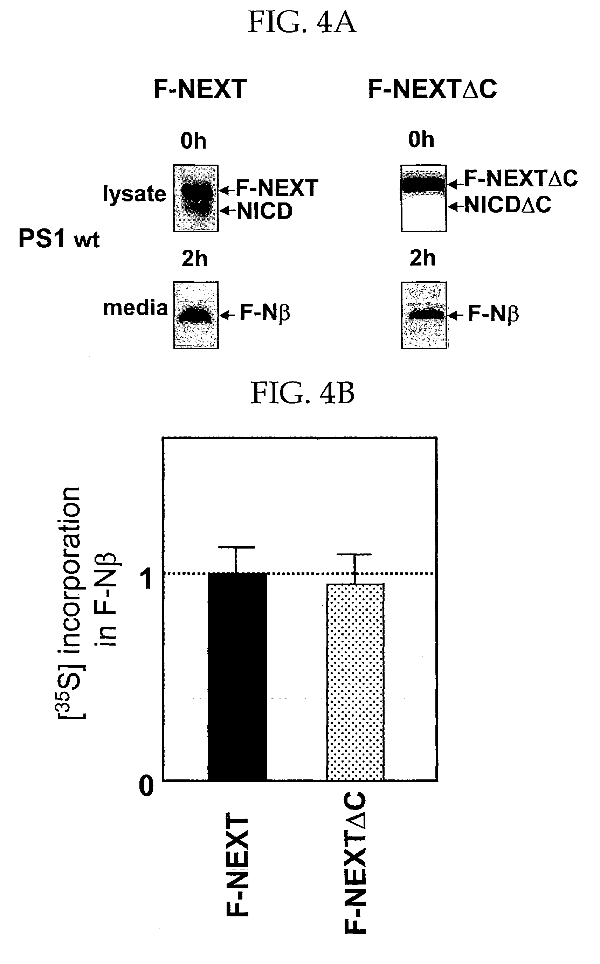

K293 cells which stably expressed F-NEXTΔC or F-NEXT were cultured in a 10 cm dish up to the confluent phase, and pulse-labeled for 30 minutes and chase-labeled for 2 hours with [³⁵S]-methionine (aforementioned). A lysate of the cells expressing F-NEXTΔC after being pulse-labeled was immunoprecipitated with 6336, separated on10 to 20% Tris-tricine SDS-PAGE, and detected by autoradiography. (FIG. 4A).

Likewise, a lysate of the cells expressing F-NEXT was treated with 9E10 antibody, and separated on 8% Tris-tricine SDS-PAGE. β-ray doses released from bands corresponding to the F-NEXTΔC and F-NEXT proteins were measured to determine the amount of the expressed proteins. As shown in FIG. 4A, it was demonstrated that almost the same amounts of the F-NEXTΔC and F-NEXT proteins were produced (FIG. 4A upper panel). Simultaneously, the secretion of radiolabeled F-Nβ was identified and quantified for the culture supernatant after being chased for 2 hours after the pulse (FIG. 4A, lower panel). The amount of secreted F-Nβ was plotted to the amount of the expressed F-NEXTΔC and F-NEXT proteins, and consequently, secretion rates of F-Nβ from the cells expressing F-NEXTΔC and F-NEXT were not different (FIG. 4B).

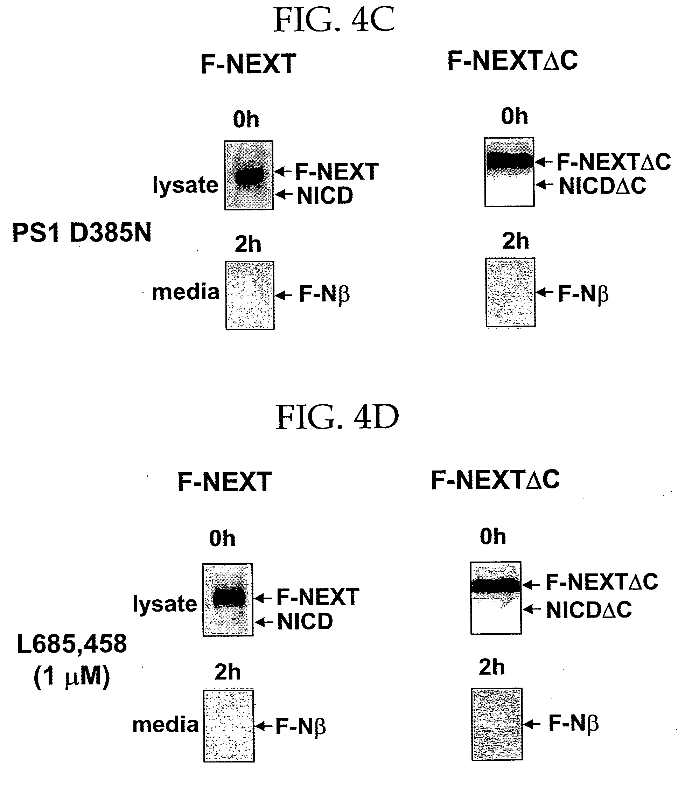

It was investigated using PS1 D385N known as the dominant negative mutation of the presenilin whether the proteolysis which we observed was the presenilin-dependent proteolysis or not. F-NEXTΔC or F-NEXT was further expressed in the cells expressing PS1 D385N, and the same experiment was performed. Consequently, although radiolabeled F-NEXTΔC or F-NEXT was present in the lysate after being pulse labeled (FIG. 4C, upper panel), no secretion of F-Nβ after the chase was observed (FIG. 4C, lower panel). This suggests that the intramembrane proteolysis of F-NEXTΔC and F-NEXT was inhibited by D385N mutation.

Furthermore, it was investigated whether the secretion of F-Nβ was inhibited or not by adding L685,458 which specifically inhibited the intramembrane proteolysis of βAPP by γ-secretase and the extracellular secretion of Aβ resulted from it to the cells expressing F-NEXTΔC or F-NEXT (FIG. 4D).

The secretion of F-Nβ from the cells expressing F-NEXTΔC or F-NEXT was almost completely inhibited by 1 µM of L685,458 (FIG. 4D lower panel). This result suggests the possibility that Aβ and F-Nβ are produced through a common mechanism (γ-secretase).

Taken together the results until here, it has been revealed at a molecular level that (1) an intramembrane proteolysis rate is not different between F-NEXTΔC and F-NEXT in terms of F-Nβ amount secreted from F-NEXTΔC and F-NEXT, and (2) this intramembrane proteolysis is caused by presenilin/γ-secretase activity which produces Aβ from βAPP.

In the case of general proteolytic enzymes, the substrate cleavage site is strictly determined, but it has been known to have its major cleavage site and its minor cleavage site group in the vicinity as the characteristic of the intramembrane proteolysis by the presenilin/γ-secretase activity. In fact, the pathogenic mutation which patients with familial Alzheimer's disease have "affects the precision of the cleavage at the major cleavage site and the minor cleavage sites in the vicinity thereof". This is believed to directly cause the disease.
The present inventors then investigated whether the precision of the S4 cleavage which produced F-Nβ was changed or not between F-NEXTΔC and F-NEXT. The precision of the S4 cleavage should be reflected to the carboxyl terminus of F-Nβ. It was investigated whether the composition of F-Nβ which was the mixture of several types produced from F-NEXTΔC or F-NEXT was the same or not. The cells expressing F-NEXTΔC or F-NEXT were cultured in a 20 cm dish up to the confluent phase, the culture supernatant was replaced and the supernatant was collected after being cultured for 3 hours. The supernatant was immunoprecipitated with M2 agarose, and subsequently molecular weight spectra of the F-Nβ mixture were depicted using an MALDI-TOF mass analyzer (FIGS. 5A-1 and 5A-2). The mass spectrometric spectra of F-Nβ secreted from the cells expressing F-NEXTΔC or F-NEXT and the molecular weights of respective molecular species were completely identical (FIGS. 5A-1, 5A-2 and 5B).

From these results, it has been suggested that the cleavage which occurs in F-NEXTΔC is identical to the intramembrane cleavage which occurs in F-NEXT, and that it does not affect the proteolysis by the presenilin/γ-secretase to drastically eliminate the amino acid sequence in the carboxyl terminal side (NICD portion) of F-NEXT (FIG. 5C).

S4 which is the intramembrane proteolytic site of Notch-1 by the presenilin/γ-secretase is composed of the major cleavage site and several minor cleavage sites in the vicinity thereof (FIG. 5C). Thus, it was investigated whether the precision of S4 cleavage of Notch-1 was not changed in the case of F-NEXTΔC obtained by remarkably modifying Notch-1 (FIG. 6A).

### (Example 3)

### An S4 cleavage pattern in the cells expressing F-NEXTΔC is extremely similar to that of Notch-1 (in vivo experiment)

Binding of Notch ligand is essential for the S2 cleavage and the subsequent intramembrane proteolysis (S3/S4). Thus, mN1-LNG CC>SS cDNA (Mumm JS, Schroeter EH, Saxena MT, Griesemer A, Tian X, Pan DJ, Ray WJ, Kopan R. A ligand-induced extracellular cleavage regulates gamma-secretase-like proteolytic activation of Notch1. Mol Cell. (2000) 5:197-206.) which was an artificial mutant of murine Notch-1 was gifted by Dr. Rafael Kopan. It has been reported that the mN1-LNG CC>SS mutant undergoes the sequential proteolysis at S2 and S3 without stimulation by binding to the ligand and NICD is released extracellularly. The present inventors incorporated this cDNA into pcDNA3.1 (hygro) (Invitrogen). K293 cells were stably transfected with the plasmid expressing this mN1-LNG CC>SS mutant.

It was investigated whether the S4 cleavage site pattern in the sequential proteolytic process of mN1-LNG CC>SS having the wild type Notch-1 sequence with no tag and the S4 cleavage pattern of F-NEXTΔC which directly became the substrate of the S4 cleavage were different or not. That is, it was investigated whether the composition of Nβ which was the mixture of several types produced from the mN1-LNG CC>SS mutant and the composition of F-Nβ produced from F-NEXTΔC were identical or not.

The cells expressing mN1-LNG CC>SS were cultured in a 20 cm dish up to the confluent phase, phorbol ester PMA was added to the culture supernatant at a concentration of 100 ng/ml, and the cells were cultured for 2 hours. Subsequently, the culture supernatant was replaced and another supernatant was collected after being cultured for 6 hours. The supernatant was immunoprecipitated with 6521, and then the molecular weight spectrum of the Nβ mixture were depicted using the MALDI-TOF mass analyzer (FIG. 6B).

The mass spectrometric spectrum pattern of the wild type Nβ secreted from the cells expressing mN1-LNG CC>SS was extremely similar to that of F-Nβ secreted from the cells expressing F-NEXTΔC (FIGS. 6B, C, 5A-1, 5A-2 and 5B).

As the above, it has been demonstrated multilaterally that the S4 cleavage of F-NEXTΔC obtained by artificially modifying the extracellular portion is not different from the S4 cleavage of murine wild type Notch-1.

### (Example 4)

It has been known that the familial Alzheimer's disease is caused by changing the precision of γ40 and γ49 intramembrane proteolytic sites of the βAPP. In order to sensitively measure the efficiency and the precision of the S3 and S4 cleavage of the Notch-1 protein which is the proteolysis by the same presenilin/γ-secretase, <a cell-free assay system which reproduced the intramembrane proteolysis of Notch-1 by the presenilin/γ-secretase> using the membrane fraction of K293 cells expressing F-NEXTΔC was made.

### -Establishment of cell-free assay system which accurately reproduces S3/S4 cleavage of Notch-1-

CTF released in the cell after the proteolysis of the βAPP protein by γ-secretase is referred to as AICD. It is difficult to identify AICD in the cell lysate whereas Aβ which is NTF is relatively easily recognized in the culture supernatant. This is because the proteolytic speed of AICD is much faster than that of Aβ. Thus, a cell-free γ-secretase assay for identifying the proteolysis which directly produces AICD in the proteolysis of the βAPP protein by the presenilin/γ-secretase has been developed (Pinnix I, Musunuru U, Tun H, Sridharan A, Golde T, Eckman C, Ziani-Cherif C, Onstead L, Sambamurti K. A novel gamma-secretase assay based on detection of the putative C-terminal fragment-gamma of amyloid beta protein precursor. J Biol. Chem. (2001) 276:481-7. McLendon C, Xin T, Ziani-Cherif C, Murphy MP, Findlay KA, Lewis PA, Pinnix I, Sambamurti K, Wang R, Fauq A, Golde TE. Cell-free assays for gamma-secretase activity. FASEB J. (2000) 14:2383-6.). And, as a result of identifying the amino terminus of AICD, the γ49 intramembrane proteolytic site was reported.

The present inventors have confirmed that F-NEXTΔC was expressed in the lysate of the cells expressing F-NEXTΔC and that simultaneously F-Nβ was released extracellularly in the culture supernatant. Subsequently, the inventors attempted to recognize NICDΔC in the cell lysate, but NICDΔC was not observed at all although 6336 recognized the expression of F-NEXTΔC (results are not shown in the figure).

After trials and errors were performed for several methods, the present inventors extracted the membrane fraction of the cells expressing F-NEXTΔC, after thoroughly washing, attempted <cell-free Notch cleavage assay (cell-free Notch cleavage analysis)>, and established the assay system (methods in FIGS. 3A and 3B, results in FIGS. 7A, 7B-1, 7B-2 and 7C).

### (Homogenizing cells and preparation of membrane fraction)

The cells expressing F-NEXTΔC were cultured in 15 of 20 cm dishes up to the confluent phase. The following process was strictly performed at 4°C. The cells were washed twice with ice-cooled PBS to exclude contamination of the culture supernatant, then collected with a cell scraper, and separated from PBS and collected by centrifugation at 1,500 x g.

Homogenization buffer (0.25 M sucrose, 10 mM HEPES, pH 7.4) and a protease inhibitor mix supplied from Roche at one time of a concentration recommended in instructions were added, and the cells were homogenized by moving a Teflon homogenizer up and down for 20 strokes. A cell homogenate was centrifuged at 1,000 x g for 5 minutes to remove a nuclear fraction and cell debris, and its supernatant (PNS) was collected. Subsequently, PNS was centrifuged at 100,000 x g for 30 minutes to collect a precipitate fraction.

This precipitate was resuspended in reaction buffer (150 mM citrate buffer, pH 6.4), the protease inhibitor mix supplied from Roche at 4 times of the concentration recommended in the instructions and 5 mM 1,10-Phenanthroline(Sigma), and centrifuged again at 100,000 x g for 30 min to wash. The precipitate after three times of this washing procedure was used as a crude membrane fraction (CMF) (FIG. 3A)

### (cell-free γ-secretase assay).

The crude membrane fraction (CMF: FIG. 3A) of the K293 cells expressing F-NEXTΔC was resuspended in the reaction buffer (150 mM citrate buffer, pH 6.4, the protease inhibitor mix supplied from Roche at 4 times of the concentration recommended in the instructions and 5 mM 1,10-Phenanthroline(Sigma)). A cell-free cleavage reaction step was made by culturing the suspension at 37°C. Here, CMF was cultured at 37°C for 40 minutes (FIG. 3B). An S1-40 min fraction (FIG. 3B) which was the supernatant obtained by centrifuging a reactant at 100,000 x g for 15 minutes was immunoprecipitated with 6336, and consequently NICDΔC was recognized. The molecular weight of this NICDΔC was analyzed using the MALDI-TOF mass analyzer (FIG. 7B-1). As a result, it was revealed that the amino terminus of NICDΔC was derived from S3 as reported (FIG. 7B-2). This is the first report for the diversity of this S3 cleavage site.

The precipitate after collecting the S1-40 min fraction was sonicated for a short time, and further centrifuged at 100,000 x g for 15 minutes to collect a supernatant fraction as an S2-40 min fraction (FIG. 3B). This S2-40 min fraction was immunoprecipitated with M2 agarose and analyzed using the MALDI-TOF mass analyzer (FIG. 7A). As a result, nearly the same mass spectrometric spectrum of F-Nβ as in the case of similarly analyzing using the culture supernatant was obtained (comparison of FIG. 7A with FIGS. 5A-1 and 5A-2). Therefore, it has been found that F-Nβ released by undergoing the cleavage by the presenilin/γ-secretase in the living cells and F-Nβ released by the cell-free Notch cleavage analysis are the mixtures of several F-Nβ molecular species with nearly the same composition.

The diversity of the S3 and S4 cleavage sites identified by the cell-free Notch cleavage analysis is illustrated in FIG. 7C.

The above results were not inconsistent with the previous study results using the living cells. The change in the diversity which is the characteristic of the S3 and S4 cleavage sites can be identified by the cell-free Notch cleavage analysis. This assay system is characterized in that by taking advantage of this, "the change in the diversity of the S3 and S4 cleavage" for the drug can be accurately investigated. For example, a part of NSAID such as ibuprofen whose possibility as a therapeutic drug for Alzheimer's disease has attracted attention affects the precision in the cleavage of βAPP by the presenilin/γ-secretase. It is possible to investigate the effect of these drugs on the cleavage of the Notch protein in detail.

The γ-secretase inhibitor has been also developed as the therapeutic drug for Alzheimer's disease, and the phase 2 trial was performed, but the development is now deadlocked due to its side effect. It is believed that this side effect is caused because the presenilin/γ-secretase mechanism is involved in the intramembrane proteolysis of over ten types of proteins including the Notch protein and the inhibitor thereof inhibits all of them. Among others, the action of the Notch protein is extremely important for the body. The system of measuring the Aβ amount in the supernatant of cells in which βAPP or the mutant thereof has been constitutively expressed has been used for screening of the presenilin/γ-secretase inhibitor which inhibits the Aβ production.

By utilizing a multiple-substrate intramembrane simultaneous assay which is one embodiment of the cell-free Notch cleavage assay of the present invention, it has been elucidated that as the nature of the γ-secretase inhibitor, "the effects on the cleavage in membrane central portion such as γ40 cleavage of βAPP and S4 cleavage of Notch and on the cleavage in the membrane portion close to cell inside such as γ49 cleavage of βAPP and S3 cleavage of Notch are different" (FIGS. 9A, 9B and FIG. 10). Therefore, it is thought that the side effect due to the inhibition of the Notch signal transduction in the screening process of the γ-secretase inhibitor is predicted before it happens, and that the drug and the concentration with no side effect can be mentioned (FIG. 10). Furthermore, it has been recently known that a part of the compounds having the NSAID effect has γ-secretase regulatory actions and affects the precision of γ40 cleavage of βAPP. Efficiencies of these compounds for other three cleavage have been investigated, but their effects on the precision was not investigated. We have recently demonstrated that the change in the precision of the S3 cleavage affects the stability of NICD produced as a result (details are not shown). Therefore, in order to prevent the side effect due to the inhibition of the signal transduction by the γ-secretase inhibitor or modifying drug, the precision of the cleavages other than γ40 which produce Aβ must be investigated when the γ-secretase inhibitor or modifying drug in a second generation will be developed. It will be essential to carry out the present patent at that time.

### (Example 5)

Next, the cell-free Notch cleavage analysis was combined with the cell-free assay system of βAPP. As a result, the system in which production efficiencies of 4 fragments, Aβ, AICD, Nβ and NICD produced from the proteolysis by the presenilin/γ-secretase could be measured simultaneously was made. This enables to screen quite new types of γ-secretase inhibitors. Because, the drugs with different profiles of the effects on four cleavages can be selected (γ-secretase inhibitors in the second generation) by separately examining the effects of the drug on the four different proteolysis which nearly simultaneously occur by the presenilin/γ-secretase.

### -Preparation of cell-free assay system which reproduces γ40/γ49 cleavages of βAPP and S3/S4 cleavages of Notch-

Human embryonic kidney 293 cells (K293 cells) in which F-NEXTΔC and Sweden type mutant βAPP (swβAPP) had been constitutively co-expressed were made by transfecting with the Sweden type mutant βAPP using Lipofectamine 2000 (Invitrogen), selecting by G418 and subsequently, transfecting the cells expressing βAPP with F-NEXTΔC. It was confirmed first that F-NEXTΔC and swβAPP had been expressed in the lysate of this cell, and simultaneously that F-Nβ and Aβ had been released in the culture supernatant (FIG. 5A to 5C). The composition of F-Nβ and Aβ molecular species were examined and it was confirmed that they were not inconsistent with previous reports (results are not shown).

Using the membrane fraction of the cells co-expressing F-NEXTΔC and swβAPP, it was attempted to establish a <multiple substrate cell-free assay system combining βAPP and Notch-1>, and a four fragments, Aβ, AICD, Nβ and NICD-producing system was established. In the present Example, L685,458 or DAPT known to be the reversible γ secretase inhibitor was used as the subject substance.

A crude purified membrane fraction of K293 cells co-expressing F-NEXTΔC and swβAPP, pretreated with 100 nM of L685,458 or 100 nM of DAPT for 2 hours before extracting the membrane fraction was used. The S1-40 min fraction was collected from this crude purified membrane fraction. The precipitate fraction after collecting the S1-40 min fraction was resuspended in the reaction buffer, and then sonicated for a short time. Subsequently the fraction was centrifuged at 100,000 x g for 15 minutes to collect the supernatant fraction as the S2-40 min fraction.

The fractions were immunoprecipitated with the antibody specific for each fragment (4G8 for Aβ, 6618 for AICD, M2 agarose for F-Nβ and 6336 for NICDΔC), and analyzed using the MALDI-TOF mass analyzer (FIG. 8). NICDΔC and AICD de novo produced were identified in the S1-40 min fraction. F-Nβ and Aβ were identified in the S2-40 min fraction (FIG. 8). Their mass spectrometric patterns were shown in FIG. 8. Even when the change was added to the multiple substrate cell-free assay system combining βAPP and Notch-1, the diversity in the proteolytic sites by the presenilin/γsecretase was conserved.

As a result of examining F-Nβ in the S2-40 min fraction, the nearly the same mass spectrometric spectrum as in the case of similarly analyzing using the culture supernatant was obtained (comparison of FIG. 8 with FIG. 5A-2).

The above polyclonal antibody (6618) for efficiently recognizing AICD which is the C terminal fragment of βAPP after the intramembrane proteolysis by the presenilin/γ-secretase is the antibody against the following synthetic polypeptide (SEQ ID NO:12), and was made as follows. First, the above polypeptide which became the antigen was prepared. The antigen was made by chemically crosslinking the Cys residue at the amino terminus of the polypeptide to the KLH protein.
SEQ ID NO:12
NH2-CKMQQNGYENPTYKFFEQMQN-COOH

### (Example 6)

### Effects of commercially available γ-secretase inhibitors (L685,458 and DAPT) on Notch-1 cleavage using multiple substrate cell-free assay system combining βAPP and Notch-1

The cells co-expressing F-NEXTΔC and swβAPP were cultured up to the confluent phase, and treated with the γ-secretase inhibitor at each concentration for 3 hours. As the γ-secretase inhibitor, L685,458 which was a transition-state analogue which binds to an active center of the presenilin/γ-secretase and DAPT which was not the transition-state analogue were used. The cells after the treatment were washed twice with ice-cooled PBS to collect. These cells were lysed and homogenated to extract CMF. Subsequently, this membrane fraction was suspended in alkaline washing buffer and left stand at 4°C for 30 minutes. Then, the resuspension and the centrifugation were repeated three times using the reaction buffer to wash out the alkaline washing buffer. It appears that most γ-secretase inhibitor contained in CMF was washed out until this process. Finally, CMF was suspended once more in the reaction buffer, and the cell-free reaction was performed. The amounts of AICD and NICDΔC in the S1-40 min fraction and the amounts of Aβ and Nβ in the S2-40 min fraction were semi-quantified from relative heights of MALDI-TOF MS peaks, and evaluated by four scales of -, +, ++ and +++.

First, the effects on the production of F-Nβ, Aβ, NICDΔC and AICD in the case of pretreating with L685,458 will be set forth. When the cells were pretreated at high concentration of 1 µM, and further 1 µM of L685,458 was added in all buffers including the homogenate buffer and the reaction buffer, all of four fragments were not observed at all. This suggests that L685,458 acted as reported in our assay system.

However, even when pretreated at high concentration of 1 µM, if thoroughly washed out, de novo production of AUCD/NICD which was γ cleaved CTF was recognized. That is, it was revealed that S3/γ49 cleavage had occurred. This suggests that L685,458 is the reversible inhibitor as reported and the bind to the presenilin/γ-secretase was dissociated by washing out. Interestingly, Aβ/Nβ which was simultaneously γ cleaved NTF was not observed at that time. This fact suggests the possibility that S4/γ40 in two cleavages which configures the γ cleavage is more sensitive to L685,458 than S3/γ49. It is also thought that it indicates that the S3/γ49 activity is not always inhibited even when the S4/γ40 activity is lowered using the presenilin/γ-secretase inhibitor.

Furthermore, the experiment was similarly performed by decreasing the concentration to 100 nM and 10 nM of L685,458 used for the pretreatment. It was revealed that de novo production of F-Nβ/Aβ which was inhibited more strongly by 1 µM L685,458 than NICDΔC/AICD was recovered along with the decrease of the concentration of the inhibitor as shown in Table. The peak heights of four fragments were maximum on average when pretreated with 10 nM of L685,458.

Subsequently, it was investigated whether the mode of action of the inhibitor affected the inhibitory profiles for these four fragments. As shown in Table, the same experiment was performed using DAPT as the inhibitor. As shown in FIGS 9A and 9B, the effects of L685,458 and DAPT on the four fragments were extremely similar. Therefore, it was revealed that the effects on the four cleavages shown by L685,458 were not the inhibitory effects specific for the transition-state analogue.
Importantly, when the effect of the inhibitor on Aβ and Nβ production was different from the effect on NICD and AICD production, the difference was conserved between substrates of βAPP and F-NEXTΔC. For example, when the pretreatment with the inhibitor inhibited the F-Nβ production more strongly than the AICD production, the F-Nβ production was inhibited more strongly than NICDΔC in F-NEXTΔC. From this, the possibility that the mode of action of the inhibitor is common in βAPP and Notch-1 has been suggested. This result was schematically represented in FIG. 10. The effect of the inhibitor on the Aβ and Nβ production is represented by a curve 1 and the effect of the inhibitor on the AICD and NICD production is represented by a curve 2.

Using this system, it was demonstrated that L685,458 and DAPT, which are available commercially and was discovered by the screening based on their effect on Aβ production, acted sensitively upon γ40 and corresponding S4 cleavage, but less sensitively on γ49 and corresponding S3 cleavage. By the use of this assay system, the possibility of the side effect expression due to Notch cleavage inhibitory effect of γ-secretase inhibitor developed as the therapeutic drug for Alzheimer's disease can be mentioned for the first time before it happens. It is thought that this assay system can be utilized for screening of the γ-secretase inhibitor in the second generation now under development.

### (Example 7)

A transgenic mouse carrying V1744G which is a mutant of the S3 cleavage site of Notch exhibits a Notch phenotype and is embryonic lethal (Huppert SS, Le A, Schroeter EH, Mumm JS, Saxena MT, Milner LA, Kopan R Embryonic lethality in mice homozygous for a processing-deficient allele of Notch1.Nature. 2000 Jun 22;405 (6789) :966-70.). In the present example, it was investigated how this mutation at S3 site was involved in the S3 and S4 cleavages.

F-NEXT V1744G and F-NEXT V1744L in which the mutation of S3 had been introduced into F-NEXT were made (FIG. 11A). After confirming the protein expression by the pulse for 30 minutes, the chase was performed for 2 hours to examine the degradation of the F-NEXT derivative (FIG.11B).

First, the efficiency of the S3 cleavage was examined. According to FIG. 11 B upper panel, it is found that F-NEXT V1744G and F-NEXT V1744L in the background of wild presenilin were degraded and their amounts were decreased during 2 hours' chase as with F-NEXT. But, according to FIG. 11B upper panel, the amounts of NICD produced from F-NEXT V1744G and F-NEXT V1744L was much smaller than that produced from F-NEXT. The similar experiment was performed in the background of PS1 D385N in which the presenilin function had been inhibited. In this case, according to FIG. 11B lower panel, not only the NICD production but also the decrease of F-NEXT and its derivatives were not observed after 2 hours' chase. The decrease of F-NEXT and its derivatives in this chase time was resulted from the intramembrane proteolysis by the presenilin.

Subsequently, the efficiency of the S4 cleavage was examined. As shown in FIG. 11C, radiolabeled F-Nβ observed in the supernatant after 2 hours' chase was not changed in F-NEXT and its derivatives F-NEXT V1744G and F-NEXT V1744L (FIG. 11C). Therefore, the efficiency of the S4 cleavage is not affected by the mutation of the S3 cleavage site. Taken together, the efficiency of the S3 and S4 cleavages is not changed by the S3 mutation, but the possibility that the intracellular amount of NICD was decreased was suggested.

In order to verify the reason for the decreased NICD amount resulted from the intramembrane proteolysis in the S3 mutant, the following experiment was performed. F-NEXTΔC and F-NEXTΔC V1744G were made in order to determine the cleavage site of F-NEXT and its S3 mutant, V1744G, by the presenilin, and the S3 cleavage site was specified by measuring the molecular weight of NICDΔC by IP-MS method after the cell-free reaction of the crude membrane fraction (FIGS. 12A-1 and 12A-2). As a result, it was revealed that the S3 cleavage site was not between G1743 and V1744 , the ordinary cleavage site, but was between G1744 and L1745, when the V1744G mutant was introduced (FIGS. 12A-1, 12A-2 and 12B). Consequently, compared with the case of F-NEXTΔC, in F-NEXTΔC V1744G, one amino acid residue is shifted at the amino terminus of produced NICDΔC. Furthermore, the stability of the NICD fragment was examined by mixing NICD extracted and purified from the cells expressing F-NEXT or F-NEXT V1744G with an intracellular fluid, and consequently, NICD in the F-NEXT V1744G cells was much more unstable than NICD in F-NEXT cells.

From the above, the followings have been found. V1744G which is the mutant at S3 cleavage site of Notch interferes with the Notch signal transduction in the mouse having the mutation. This cause was examined in detail. Although the cleavage efficiency at S3 and S4 cleavage sites was not decreased by this mutation, the intracellular NICD amount was decreased. This appears to be caused because the S3 cleavage site was shifted by this mutation, consequently the amino acid residue at the amino terminus of NCID was changed and the stability of NCID itself was lowered. This result suggests that the change in the precision of the S3 cleavage decreased the signal transduction quantity.

### (Example 8)

### Mass spectrometry of compounds

An inhibitory effect of Aβ42 production and an augmentation effect of Aβ38 production were observed using the derivative of NSAID as the subject substance by the cell-free assay. The effects were observed by detecting using the IP-MS method after the cell-free reaction in the Notch and βAPP co-expression system. The cells were treated with the subject substance for 24 hours before collecting the crude membrane fraction, and the subject substance was added in all media and buffers until completing the experiment. As a result, the increase of Aβ38 production along with the decrease of Aβ42 production was observed (FIG. 13). No effect of this drug on the amino terminus of AICD was observed (FIG. 13). Furthermore, this effect was not observed for Notch (FIG.13).

### Industrial Applicability

The present invention can be utilized for drug screening of γ-secretase inhibitors effective for the treatment of Alzheimer's disease and the production of composition used therefor.

## Claims

1. A method of analyzing cell-free Notch cleavage comprising:
a cell-free cleavage reaction step of contacting a subject substance with a crude membrane fraction of a cell in which a Notch protein mutant has been expressed; and
a detection step of detecting a fragment of an amino terminal side and a fragment of a carboxyl terminal side resulted from intramembrane proteolysis of the Notch protein mutant in said cell-free cleavage reaction step,
wherein an effect of the subject substance on the intramembrane proteolysis of the Notch protein mutant is analyzed, and
said Notch protein mutant has at least a putative transmembrane domain of a Notch protein, deletes a part of an amino acid sequence in the amino terminal side than at least the transmembrane domain, and has antibody recognition sites in the amino terminal side than the transmembrane domain and the carboxyl terminal side, respectively.

2. The method for analyzing the cell-free Notch cleavage according to claim 1, wherein the effect of subject substance on an amount of the intramembrane proteolysis of the Notch protein mutant is analyzed in said detection step.

3. The method for analyzing the cell-free Notch cleavage according to claim 1, wherein said detection step is a step of detecting the fragment of said amino terminal side and the fragment of said carboxy terminal side using a mass spectrometry, and the effect of the subject substance on site precision of the intramembrane proteolysis of the Notch protein mutant is analyzed.

4. The method for analyzing the cell-free Notch cleavage according to any of claims 1 to 3, wherein the crude membrane fraction is a crude membrane fraction of a cell in which the Notch protein mutant and a βAPP protein mutant have been co-expressed, the detection step further has a detection step of detecting a fragment of the amino terminal side and a fragment of the carboxyl terminal side resulted from intramembrane proteolysis of the βAPP protein mutant in said cell-free cleavage reaction step, wherein an effect of the subject substance on the intramembrane proteolysis of the Notch protein mutant and βAPP protein mutant are analyzed, and said βAPP protein mutant has at least a putative transmembrane domain of a βAPP protein and has antibody recognition sites at the amino terminal side than the putative transmembrane domain and the carboxyl terminal side, respectively.

5. The method for analyzing the cell-free Notch cleavage according to any of claims 1 to 4, wherein the Notch protein mutant is either (1) or (2):
(1) Polypeptide (FLAG-NEXT) represented by SEQ ID NO:1; 
SEQ ID NO:1;
MPRLLTPLLCLTLLPARAARGLRDYKDDDDKMVMKSEPVEPPLPS
QLHLMYVAAAAFVLLFFVGCGVLLSRKRRRQHGQLWFPEGFKVSE
ASKKKRREPLGEDSVGLKPLKNASDGALMDDNQNEWGDEDLETK
KFRFEEPVVLPDLSDQTDHRQWTQQHLDAADLRMSAMAPTPPQG
EVDADCMDVNVRGPDGFTPLMIASCSGGGLETGNSEEEEDAPAVIS
DFIYQGASLHNQTDRTGETALHLAARYSRSDRRKRLEASADANIQ
DNMGRTPLHAAVSADAQGVFQILLRNRATDLDARMHDGTTPLIL
AARLAVEGMLEDLINSHADVNAVDDLGKSALHWAAAVNNVDA
AVVLLKNGANKDIENNKEETSLFLSIRRESYETAKVLLDHFANRDIT
DHMDRLPRDIAQERMHHDIVRLLDEYNLVRSPQLHGTALGGTPTL
SPTLCSPNGYPGNLKSATQGKKARKPSTKGLACGSKEAKDLKARR
KSSQDGKGWLLDSSEQKLISEEDLEQKLISEEDLEQKLISEEDLEQKLI
SEEDLEQKLISEEDLEQKLISEEDL
(2) A polypeptide in which tendencies of sites and amounts of the intramembrane proteolysis by γ-secretase are substantially the same as in the polypeptide (FLAG-NEXT) represented by SEQ ID NO:1.

6. The method for analyzing the cell-free Notch cleavage according to claim 5, wherein the Notch protein mutant is said polypeptide of (2) and mass spectrometry in the Notch protein mutant is possible when the polypeptide according to said (2) has been cleaved in the cell-free cleavage reaction step.

7. The method for analyzing the cell-free Notch cleavage according to any of claims 5 and 6, wherein the Notch protein mutant is a polypeptide having a deletion of a part of an amino acid sequence at a carboxyl terminal side than the putative transmembrane domain in the polypeptide (FLAG-NEXT) represented by SEQ ID NO:1.

8. A method for screening drugs comprising:
selecting an active component of the drug by using as an indicator at least any of an increase/ decrease of cleaved Notch amount and change of precision of Notch cleavage sites by a subject substance, detected by the method for analyzing the cell-free Notch cleavage according to any of claims 1 to 7.

9. A recombinant protein comprising:
wherein the recombinant protein is either one of a polypeptide; a polypeptide (FLAG-NEXTΔC) represented by SEQ ID NO:2, and a polypeptide composed of an amino acid sequence having deletion, substitution or addition of one or more amino acid residue in the amino acid sequence of said FLAG-NEXTΔC and where tendencies of cleavage sites and cleavage amounts by γ-secretase are substantially the same as in FLAG-NEXTΔC.

SEQ ID NO:2;
MPRLLTPLLCLTLLPARAARGLRDYKDDDDKMVMKSEPVEPPLPS

QLHLMYVAAAAFVLLFFVGCGVLLSRKRRRQHGQLWFPEGFKVSE
AEQKLISEEDL

10. A gene for expression comprising:
wherein the gene for expression is encoding the recombinant protein according to claim 9.

11. A recombinant vector comprising:
a gene encoding the recombinant protein according to claim 9.

12. A transformant comprising:
the recombinant vector containing a gene encoding the recombinant protein according to claim 9.
